# EUROPEAN PATENT APPLICATION

(11) **EP 4 299 005 A2**
(43) Date of publication of application: **03.01.2024**
(21) Application number: 23210973.6
(22) Date of filing: 12.04.2019
(51) Int. Cl.: A61B 5/153

(54) **BIOLOGICAL FLUID COLLECTION SYSTEM**

(30) Priority: 17.04.2018 US 201862658737 P
(62) Divisional of application: 19720274.0
(71) Applicant: Becton, Dickinson and Company, Franklin Lakes, NJ 07417 (US)
(72) Inventor: IVOSEVIC, Milan, Kinnelon, 07405 (US); EDELHAUSER, Adam, Kinnelon, 07405 (US); TORRIS, Anthony, V., Montclair, 07042 (US); WILKINSON, Bradley, M., Haledon, 07508 (US); CRAWFORD, Jamieson, W., 12938 Hagersten (SE)
(74) Representative: dompatent von Kreisler Selting Werner - Partnerschaft von Patent- und Rechtsanwälten mbB

(57) **Abstract**

A biological fluid collection system that includes a power source for a collection module that receives a sample and provides flow-through blood stabilization technology and a precise sample dispensing function for point-of-care and near patient testing applications is disclosed.

## Description

### CROSS-REFERENCE TO RELATED APPLICATION

The present application claims priority to United States Provisional Application Serial No. 62/658,737 entitled "Biological Fluid Collection System", filed April 17, 2018, the entire disclosure of which is hereby incorporated by reference in its entirety.

### BACKGROUND OF THE INVENTION

### 1. Field of the Disclosure

The present disclosure relates generally to a biological fluid collection system. More particularly, the present disclosure relates to a power source for a collection module for collecting a small sample of blood and dispensing a portion of the sample into a device for analyzing the sample such as a point-of-care or a near-patient-testing device.

### 2. Description of the Related Art

A need exists for a device which enables collection of a micro-sample, such as less than 500 microliters of collected sample for analysis, for patient point-of-care applications. Current devices require conventional sample collection and the subsequent use of a 1 ml syringe or pipette to transfer a small blood sample to a point-of-care cartridge or instrument receiving port. Such an open system approach results in an increased blood exposure risk for personnel performing the testing, as well as the collection of excess specimen required for a specified test procedure.

It is therefore desirable to have a blood sample collection and dispensing tool for point-of-care applications which incorporates conventional automatic blood draw and includes a novel controlled sample dispensing capability while minimizing exposure risk.

### SUMMARY OF THE INVENTION

The present disclosure provides a biological fluid collection system that includes a power source for a collection module that receives a sample and provides flow-through blood stabilization technology and a precise sample dispensing function for point-of-care and near patient testing applications.

In accordance with an embodiment of the present invention, a biological fluid collection system includes a collection module adapted to receive a sample, the collection module comprising a housing having an inlet port and an outlet port, the inlet port and the outlet port in fluid communication; a mixing chamber disposed between the inlet port and the outlet port; and a collection chamber disposed between the mixing chamber and the outlet port, the collection chamber including an actuation portion, wherein the actuation portion is transitionable between a first position in which the sample is containable within the collection chamber and a second position in which a portion of the sample is expelled from the collection chamber; and a power source removably connectable with the collection module, the power source creates a vacuum that draws the sample within the collection chamber, the power source comprising a barrel in communication with the collection chamber, the barrel defining an interior and having a first end, a second end, and a sidewall therebetween; a piston slidably disposed within the interior of the barrel, the piston sized relative to the interior to provide sealing engagement with the sidewall of the barrel, the piston transitionable between a first piston position, in which the piston is a first distance from the first end of the barrel, and a second piston position, in which the piston is a second distance from the first end of the barrel, the second distance greater than the first distance; and a spring disposed between the first end of the barrel and the piston.

In one configuration, the power source includes an activation button disposed on a portion of the barrel; and a lock in communication with the spring and the activation button, the lock transitionable between a locked position, in which the lock locks the piston in the first piston position and maintains the spring in a compressed position, and an unlocked position, in which the piston is unlocked and the spring is permitted to drive the piston to the second piston position thereby creating a vacuum that draws the sample within the collection chamber, wherein actuation of the activation button moves the lock to the unlocked position. In another configuration, the barrel is removably connectable with a portion of the collection module. In yet another configuration, the collection module includes a sample stabilizer disposed between the inlet port and the mixing chamber; and a cap having a venting plug, the cap seals the outlet port, wherein the venting plug allows air to pass therethrough and prevents the sample from passing therethrough. In one configuration, the biological fluid collection system includes a material including pores disposed between the inlet port and the mixing chamber; and a dry anticoagulant powder within the pores of the material. In another configuration, the sample dissolves and mixes with the dry anticoagulant powder while passing through the material. In yet another configuration, the material is an open cell foam. In one configuration, the sample stabilizer is the dry anticoagulant powder. In another configuration, the biological fluid collection system includes a closure covering the inlet port. In yet another configuration, the sample is a blood sample.

In accordance with another embodiment of the present invention, a biological fluid collection system includes a collection module adapted to receive a sample, the collection module comprising a housing having an inlet port and an outlet port, the inlet port and the outlet port in fluid communication; a mixing chamber disposed between the inlet port and the outlet port; and a collection chamber disposed between the mixing chamber and the outlet port, the collection chamber including an actuation portion, wherein the actuation portion is transitionable between a first position in which the sample is containable within the collection chamber and a second position in which a portion of the sample is expelled from the collection chamber; and a power source removably connectable with the collection module, the power source having a vacuum that draws the sample within the collection chamber, the power source comprising a spike in communication with the collection chamber; an evacuated tube having a first tube end, a second tube end, and a sidewall extending therebetween and defining a tube interior, the evacuated tube containing the vacuum; and a closure sealing the first tube end, wherein, with the evacuated tube engaged with the spike such that a portion of the spike pierces the closure and enters the tube interior, the vacuum of the evacuated tube draws the sample within the collection chamber.

In one configuration, the power source includes a tube holder removably connectable with a portion of the collection module, the tube holder defining an interior and having a first end, a second end, and a tube holder sidewall therebetween. In another configuration, the evacuated tube is movably disposed within the interior of the tube holder between a first tube position, in which the evacuated tube is disengaged from the spike, and a second tube position, in which the closure of the evacuated tube is pierced by the spike. In yet another configuration, with the evacuated tube in the first tube position, a portion of the second tube end is exposed from the second end of the tube holder and the second tube end can be pushed to move the evacuated tube to the second tube position. In one configuration, the second tube end comprises an arcuate surface. In another configuration, the collection module includes a sample stabilizer disposed between the inlet port and the mixing chamber; and a cap having a venting plug, the cap seals the outlet port, wherein the venting plug allows air to pass therethrough and prevents the sample from passing therethrough. In yet another configuration, the biological fluid collection system includes a material including pores disposed between the inlet port and the mixing chamber; and a dry anticoagulant powder within the pores of the material. In one configuration, the sample dissolves and mixes with the dry anticoagulant powder while passing through the material. In another configuration, the material is an open cell foam. In yet another configuration, the sample stabilizer is the dry anticoagulant powder. In one configuration, the biological fluid collection system includes a collection module closure covering the inlet port. In another configuration, the sample is a blood sample.

In accordance with another embodiment of the present invention, a biological fluid collection system includes a collection module adapted to receive a sample, the collection module comprising a housing having an inlet port and an outlet port, the inlet port and the outlet port in fluid communication; a mixing chamber disposed between the inlet port and the outlet port; and a collection chamber disposed between the mixing chamber and the outlet port, the collection chamber including an actuation portion, wherein the actuation portion is transitionable between a first position in which the sample is containable within the collection chamber and a second position in which a portion of the sample is expelled from the collection chamber; and a power source removably connectable with the collection module, the power source creates a vacuum that draws the sample within the collection chamber, the power source comprising a barrel in communication with the collection chamber, the barrel defining an interior and having a first end, a second end, and a sidewall therebetween; a stopper slidably disposed within the interior of the barrel, the stopper sized relative to the interior to provide sealing engagement with the sidewall of the barrel, the stopper transitionable between a first stopper position, in which the stopper is a first distance from the first end of the barrel, and a second stopper position, in which the stopper is a second distance from the first end of the barrel, the second distance greater than the first distance; and a plunger having a first plunger end and a second plunger end, a portion of the first plunger end engaged with the stopper, wherein movement of the plunger away from the first end of the barrel moves the stopper to the second stopper position thereby creating a vacuum that draws the sample within the collection chamber.

In one configuration, the barrel is removably connectable with a portion of the collection module. In another configuration, the collection module includes a sample stabilizer disposed between the inlet port and the mixing chamber; and a cap having a venting plug, the cap seals the outlet port, wherein the venting plug allows air to pass therethrough and prevents the sample from passing therethrough. In yet another configuration, the biological fluid collection system includes a material including pores disposed between the inlet port and the mixing chamber; and a dry anticoagulant powder within the pores of the material. In one configuration, the sample dissolves and mixes with the dry anticoagulant powder while passing through the material. In another configuration, the material is an open cell foam. In yet another configuration, the sample stabilizer is the dry anticoagulant powder. In one configuration, the biological fluid collection system includes a closure covering the inlet port. In another configuration, the sample is a blood sample.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
**Fig. 1** is a cross-sectional side elevation view of a biological fluid collection system with a lock in a locked position in accordance with an embodiment of the present invention.
**Fig. 2** is a cross-sectional side elevation view of a biological fluid collection system with a lock in an unlocked position in accordance with an embodiment of the present invention.
**Fig. 3** is a cross-sectional side elevation view of a biological fluid collection system with a collection module disconnected from a power source in accordance with an embodiment of the present invention.
**Fig. 4A** is a perspective view of a power source in accordance with an embodiment of the present invention.
**Fig. 4B** is a cross-sectional side elevation view of a power source in accordance with an embodiment of the present invention.
**Fig. 5A** is a perspective view of a biological fluid collection system in accordance with another embodiment of the present invention.
**Fig. 5B** is an exploded view of a biological fluid collection system in accordance with another embodiment of the present invention.
**Fig. 5C** is a side elevation view of a biological fluid collection system in accordance with another embodiment of the present invention.
**Fig. 5D** is a cross-sectional view taken along line **5D-5D** of **Fig. 5C** in accordance with another embodiment of the present invention.
**Fig. 5E** is a side elevation view of a biological fluid collection system in accordance with another embodiment of the present invention.
**Fig. 5F** is a cross-sectional view taken along line **5F-5F** of **Fig. 5E** in accordance with another embodiment of the present invention.
**Fig. 6A** is a cross-sectional side elevation view of a power source with a lock in a locked position in accordance with another embodiment of the present invention.
**Fig. 6B** is a cross-sectional side elevation view of a power source with a lock in an unlocked position in accordance with another embodiment of the present invention.
**Fig. 6C** is a cross-sectional side elevation view of a power source with a lock in an unlocked position in accordance with another embodiment of the present invention.
**Fig. 7A** is a perspective view of a biological fluid collection system in accordance with another embodiment of the present invention.
**Fig. 7B** is a cross-sectional, exploded view of a biological fluid collection system in accordance with another embodiment of the present invention.
**Fig. 7C** is a side elevation view of a biological fluid collection system in accordance with another embodiment of the present invention.
**Fig. 7D** is a cross-sectional view taken along line **7D-7D** of **Fig. 7C** with a lock in a locked position in accordance with another embodiment of the present invention.
**Fig. 7E** is a cross-sectional side elevation view of a power source with a lock in an unlocked position in accordance with another embodiment of the present invention.
**Fig. 8A** is a perspective view of a biological fluid collection system in accordance with another embodiment of the present invention.
**Fig. 8B** is a perspective, exploded view of a biological fluid collection system in accordance with another embodiment of the present invention.
**Fig. 8D** is a side elevation view of a biological fluid collection system in accordance with another embodiment of the present invention.
**Fig. 8E** is a cross-sectional view taken along line **8E-8E** of **Fig. 8D** in accordance with another embodiment of the present invention.
**Fig. 9** is a cross-sectional side elevation view of a biological fluid collection system with an evacuated tube in a first tube position in accordance with another embodiment of the present invention.
**Fig. 10** is a cross-sectional side elevation view of a biological fluid collection system with an evacuated tube in a second tube position in accordance with another embodiment of the present invention.
**Fig. 11** is a cross-sectional side elevation view of a biological fluid collection system with a collection module disconnected from a power source in accordance with another embodiment of the present invention.
**Fig. 12A** is a perspective view of a biological fluid collection system in accordance with another embodiment of the present invention.
**Fig. 12B** is an exploded view of a biological fluid collection system in accordance with another embodiment of the present invention.
**Fig. 12C** is a side elevation view of a biological fluid collection system in accordance with another embodiment of the present invention.
**Fig. 12D** is a cross-sectional view taken along line **12D-12D** of **Fig. 12C** with an evacuated tube in a second tube position in accordance with another embodiment of the present invention.
**Fig. 12E** is a cross-sectional side elevation view of a power source with an evacuated tube in a first tube position in accordance with another embodiment of the present invention.
**Fig. 13A** is a perspective view of a power source in accordance with another embodiment of the present invention.
**Fig. 13B** is a perspective, exploded view of a power source in accordance with another embodiment of the present invention.
**Fig. 14A** is a perspective view of a biological fluid collection system in accordance with another embodiment of the present invention.
**Fig. 14B** is a side elevation view of a biological fluid collection system in accordance with another embodiment of the present invention.
**Fig. 14C** is a cross-sectional view taken along line **14C-14C** of **Fig. 14B** in accordance with another embodiment of the present invention.
**Fig. 15** is a cross-sectional side elevation view of a biological fluid collection system with a stopper in a first stopper position in accordance with another embodiment of the present invention.
**Fig. 16** is a cross-sectional side elevation view of a biological fluid collection system with a stopper in a second stopper position in accordance with another embodiment of the present invention.
**Fig. 17** is a cross-sectional side elevation view of a collection module in accordance with another embodiment of the present invention.
**Fig. 18** is a cross-sectional perspective view of a collection module with a deformable portion in an initial position adjacent a point-of-care testing device in accordance with an embodiment of the present invention.
**Fig. 19** is a cross-sectional perspective view of a collection module with a deformable portion in a deformed position adjacent a point-of-care testing device in accordance with an embodiment of the present invention.
**Fig. 20** is a perspective view of an open cell foam material in accordance with an embodiment of the present invention.
**Fig. 21** is a microscopic view of the microstructure of an open cell foam material having a dry anticoagulant powder distributed throughout its microstructure in accordance with an embodiment of the present invention.
**Fig. 22** is a cross-sectional side elevation view of a collection module with a cap in accordance with an embodiment of the present invention.
**Fig. 23** is a cross-sectional side elevation view of a collection module with a deformable portion in an initial position in accordance with an embodiment of the present invention.
**Fig. 24** is a cross-sectional side elevation view of a collection module with a deformable portion in a deformed position in accordance with an embodiment of the present invention.
**Fig. 25** is a perspective view of a collection module in accordance with an embodiment of the present invention.
**Fig. 26** is a perspective view of a cap being removed from a collection module in accordance with an embodiment of the present invention.
**Fig. 27** is a perspective view of a biological fluid collection system inserted into a tube holder in accordance with an embodiment of the present invention.
**Fig. 28** is a cross-sectional view of a biological fluid collection system inserted into a tube holder in accordance with an embodiment of the present invention.
**Fig. 29** is a perspective view of a biological fluid collection system being removed from a tube holder in accordance with an embodiment of the present invention.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

The present disclosure provides a biological fluid collection system that includes a power source for a collection module that receives a sample and provides flow-through blood stabilization technology and a precise sample dispensing function for point-of-care and near patient testing applications. A collection module of the present disclosure is able to effectuate distributed mixing of a sample stabilizer within a blood sample and dispense the stabilized sample in a controlled manner. In this manner, a biological fluid collection system of the present disclosure enables blood micro-sample management, e.g., passive mixing with a sample stabilizer and controlled dispensing, for point-of-care and near patient testing applications.

Advantageously, a biological fluid collection system of the present disclosure provides a consistent blood sample management tool for point-of-care and near patient testing applications, automatic blood draw, passive mixing technology, and controlled small sample dispensing capability to point-of-care cartridge and standard luer interfaces with near patient testing receiving ports.

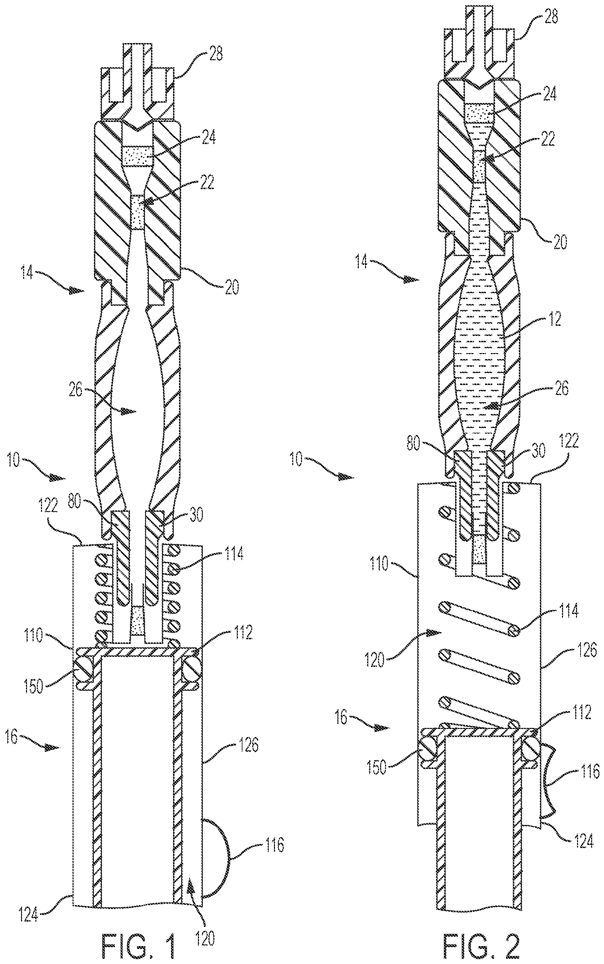

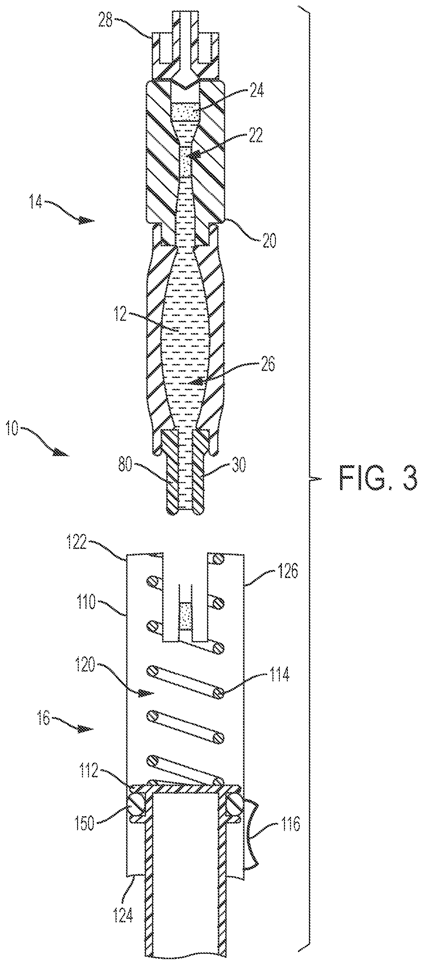

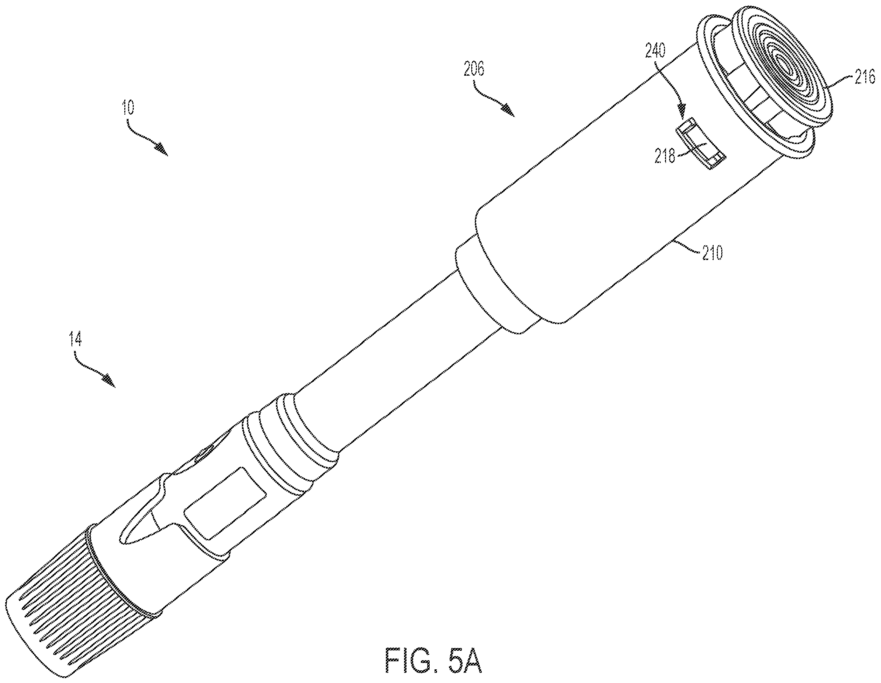

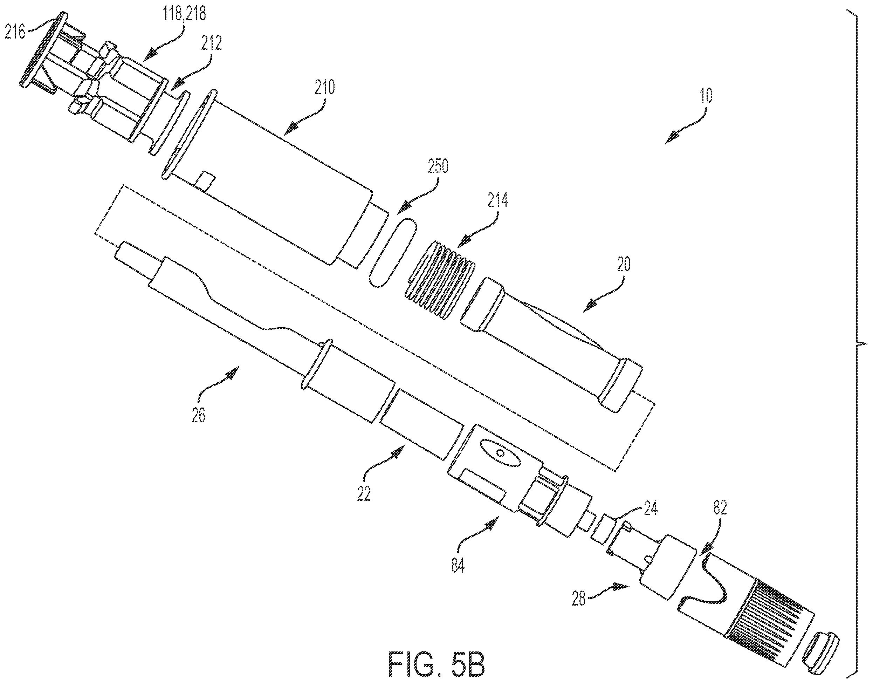

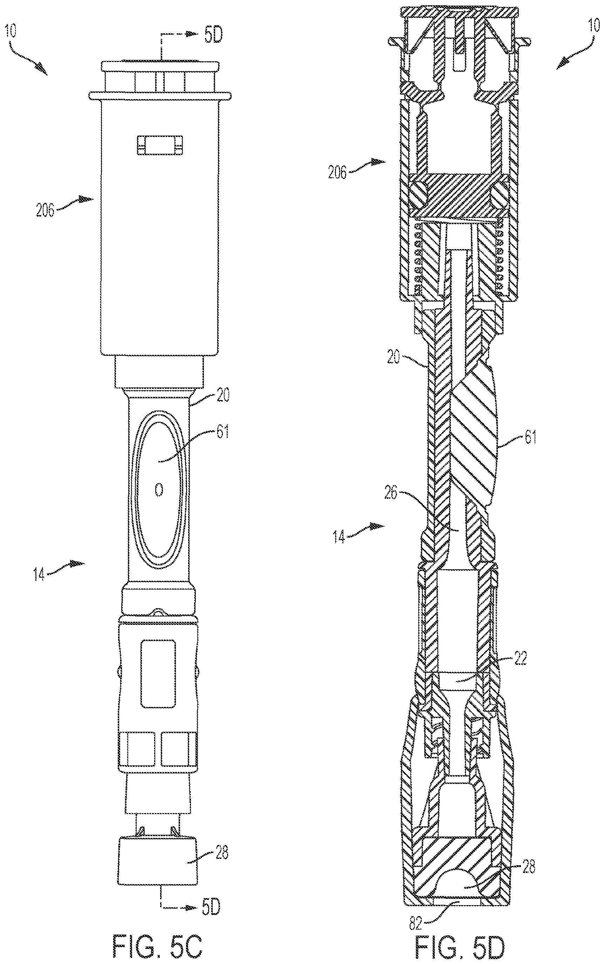

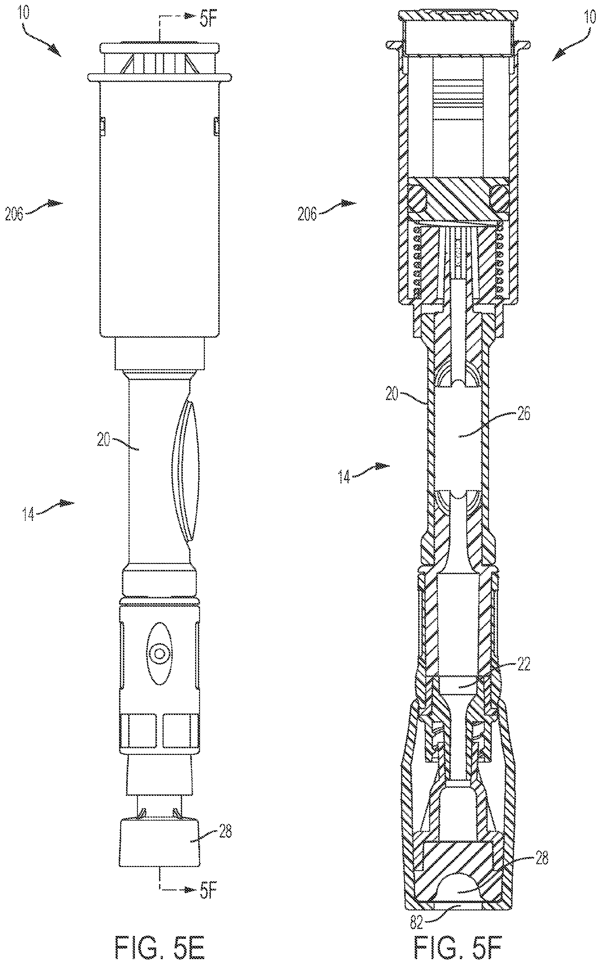

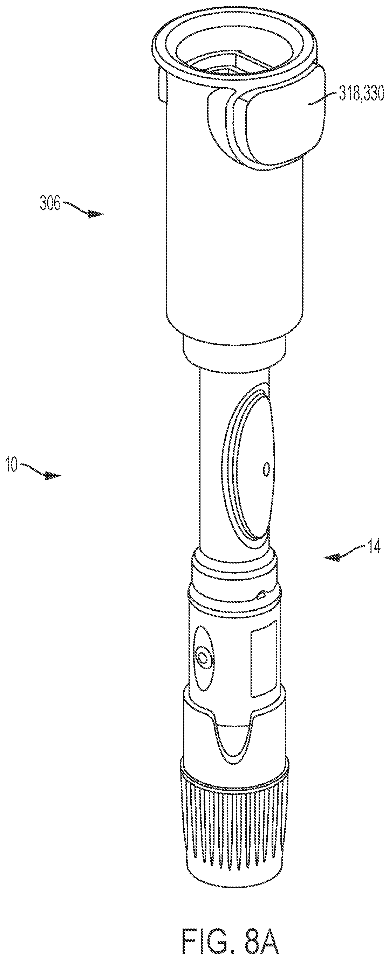

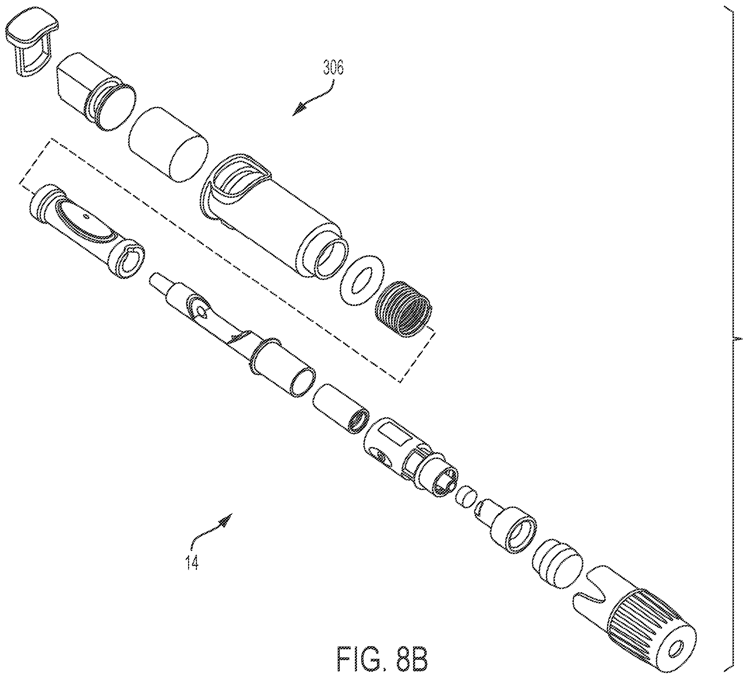

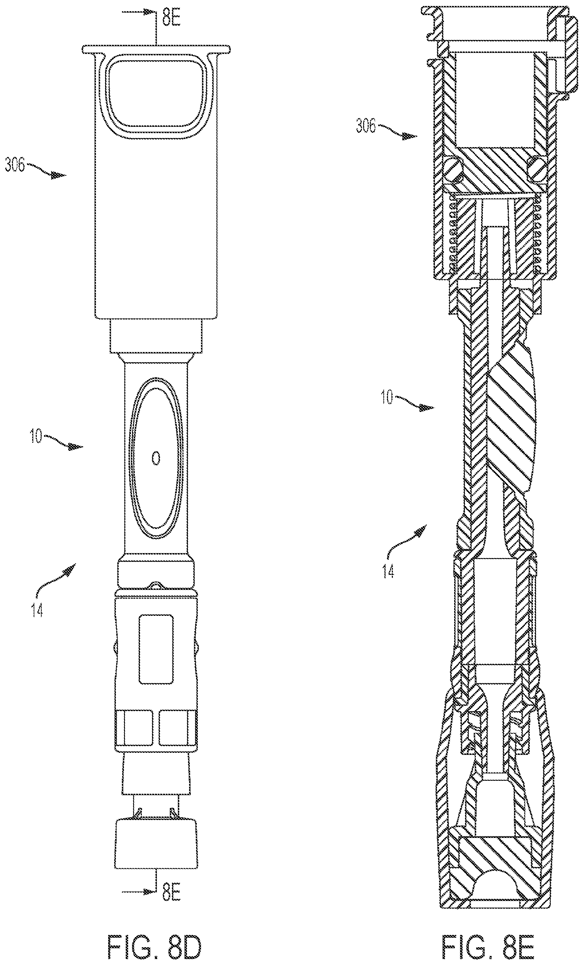

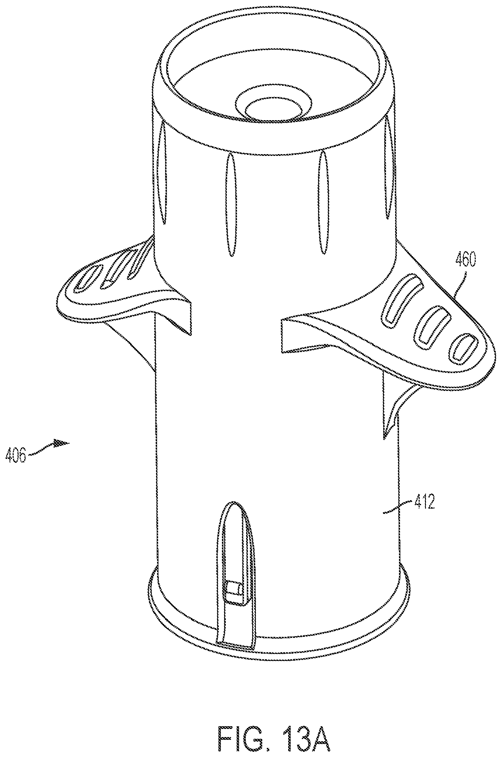

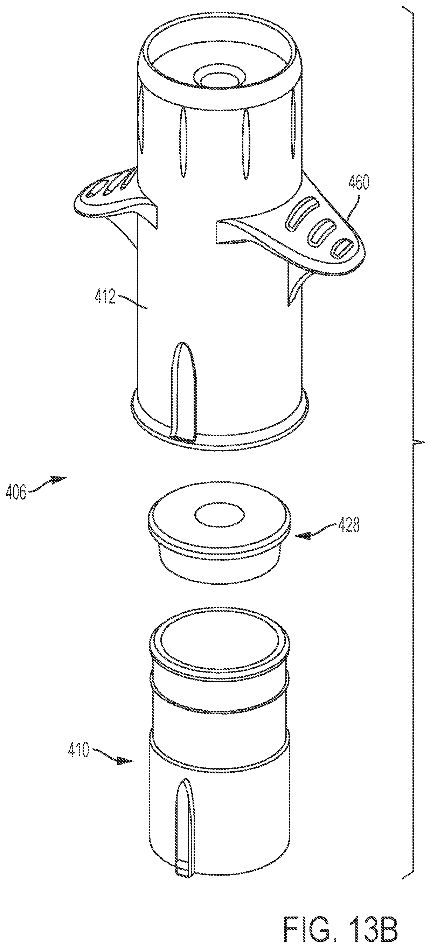

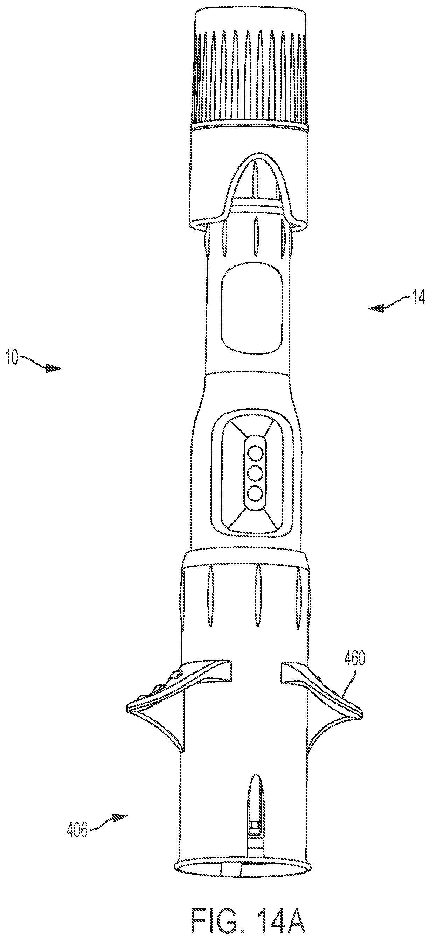

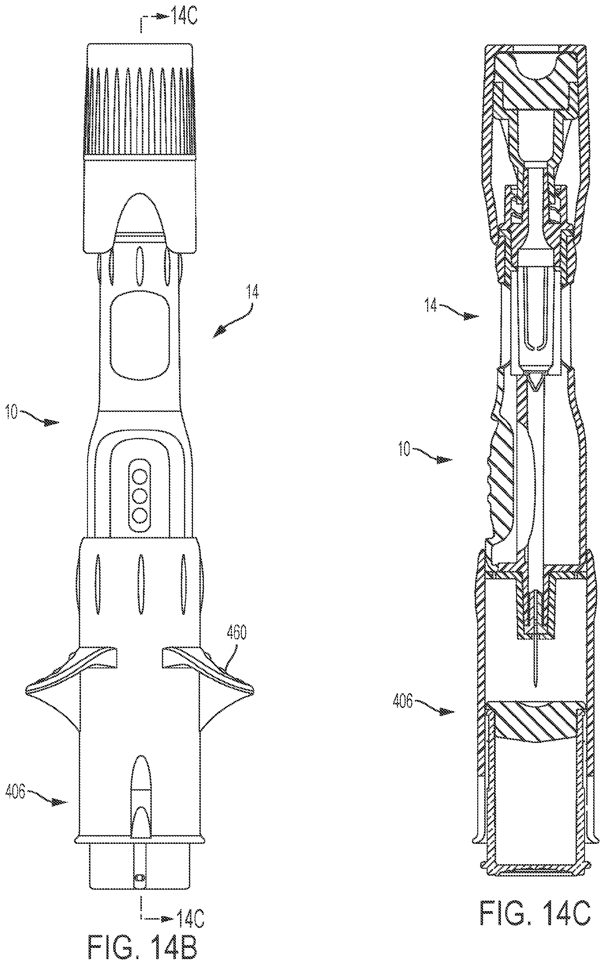

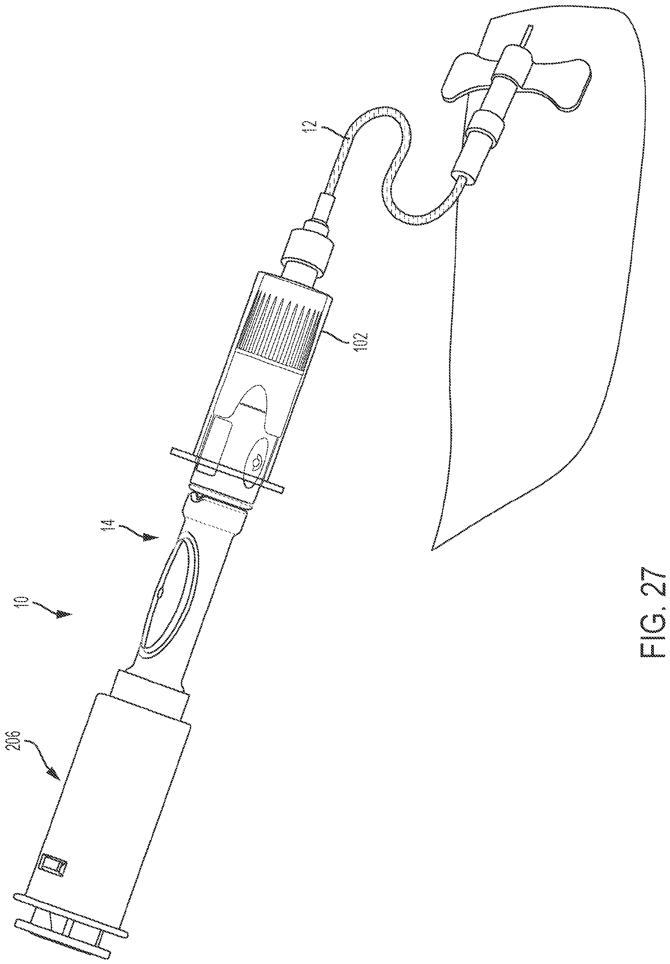

Figs. 1-29 illustrate exemplary embodiments of a biological fluid collection system 10 of the present disclosure that is adapted to receive a biological fluid sample, such as a blood sample 12. In one embodiment, the biological fluid collection system 10 of the present disclosure includes a collection module 14 that is adapted to receive a blood sample 12 and a power source 16 that is removably connectable with the collection module 14. A power source of the present disclosure provides a user activated vacuum source for drawing a biological fluid sample within a collection module 14.

Referring to Figs. 1-3, 9-11, 15-19, and 22-29, in one embodiment, the collection module 14 of the present disclosure is adapted to receive a biological fluid sample, such as a blood sample 12, and includes a housing 20, a mixing chamber 22, a sample stabilizer 24, a collection chamber 26, a closure 28, and a cap 30.

In one embodiment, the housing 20 of the collection module 14 includes an inlet port 32 and an outlet port 34. The inlet port 32 and the outlet port 34 are in fluid communication via a passageway 36 extending therebetween.

The mixing chamber 22 and the collection chamber 26 are provided in fluid communication with the passageway 36. The mixing chamber 22 and the collection chamber 26 are positioned such that a biological fluid sample, such as a blood sample 12, introduced into the inlet port 32 of the collection module 14 will first pass through a sample stabilizer 24, then the blood sample 12 and the sample stabilizer 24 pass through the mixing chamber 22, and subsequently the sample 12 with the sample stabilizer 24 properly mixed therein flow into the collection chamber 26, prior to reaching the outlet port 34 of the collection module 14. In this way, the blood sample 12 may be mixed with a sample stabilizer 24, such as an anticoagulant or other additive, provided within the collection module 14, before passing through the mixing chamber 22 for proper mixing of the sample stabilizer 24 within the blood sample 12, and then the stabilized sample is received and stored within the collection chamber 26.

In one embodiment, a sample stabilizer 24 is disposed between the inlet port 32 and the mixing chamber 22. The collection module 14 of the present disclosure provides passive and fast mixing of a blood sample 12 with the sample stabilizer 24. For example, the collection module 14 includes a mixing chamber 22 that allows for passive mixing of the blood sample 12 with an anticoagulant or another additive, such as a blood stabilizer, as the blood sample 12 flows through the mixing chamber 22.

The sample stabilizer can be an anticoagulant, or a substance designed to preserve a specific element within the blood such as, for example, RNA, protein analyte, or other element. In one embodiment, the sample stabilizer 24 is disposed between the inlet port 32 and the mixing chamber 22. In other embodiments, the sample stabilizer 24 may be disposed in other areas within the housing 20 of the collection module 14.

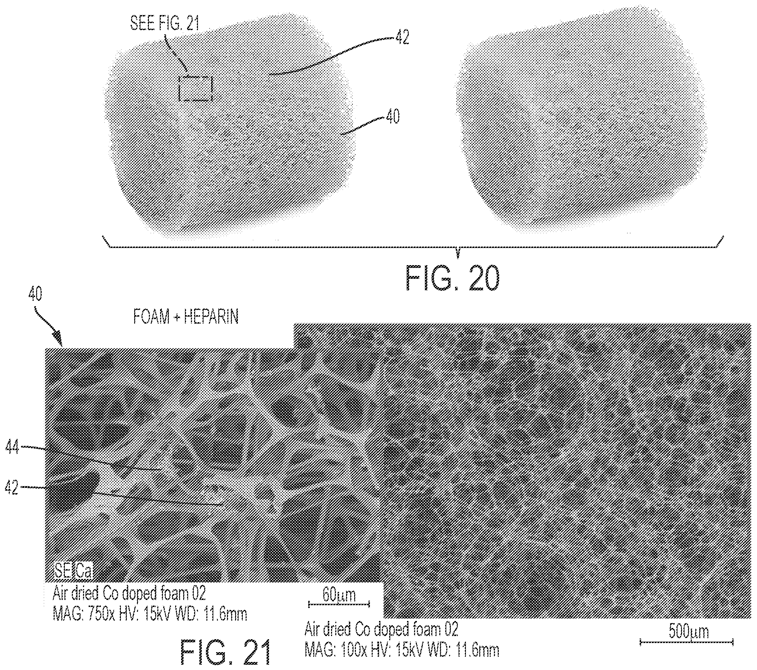

Referring to Figs. 20-23, in one embodiment, the collection module 14 includes a material 40 including pores 42 that is disposed between the inlet port 32 and the mixing chamber 22 and a dry anticoagulant powder 44 that is within the pores 42 of the material 40. In this manner, the collection module 14 may include a dry anticoagulant, such as Heparin or EDTA, deposited on or within a portion of the collection module 14. In one embodiment, the material 40 is an open cell foam that contains dry anticoagulant dispersed within the cells of the open cell foam to promote the effectiveness of the flow-through mixing and anticoagulant uptake. In one embodiment, the sample stabilizer 24 is the dry anticoagulant powder 44.

In one embodiment, the open cell foam may be treated with an anticoagulant to form a dry anticoagulant powder finely distributed throughout the pores of the open cell foam. As the blood sample 12 enters the collection module 14, the blood sample 12 passes through the open cell foam and is exposed to the anticoagulant powder available throughout the internal pore structure of the open cell foam. In this manner, the sample 12 dissolves and mixes with the dry anticoagulant powder 44 while passing through the material 40 or open cell foam.

The open cell foam may be a soft deformable open cell foam that is inert to blood, for example, a melamine foam, such as Basotect^{®} foam commercially available from BASF, or may consist of a formaldehyde-melamine-sodium bisulfite copolymer. The open cell foam may also be a flexible, hydrophilic open cell foam that is substantially resistant to heat and organic solvents. In one embodiment, the foam may include a sponge material.

The anticoagulant or other additive may be introduced into the open cell foam by soaking the foam in a liquid solution of the additive and water and subsequently evaporating the water forming a dry additive powder finely distributed throughout the internal structure of the foam.

The collection module 14 includes a mixing chamber 22 that allows for passive mixing of the blood sample 12 with an anticoagulant or another additive, such as a blood stabilizer, as the blood sample 12 flows through the mixing chamber 22. In one embodiment, the mixing chamber 22 is disposed between the inlet port 32 and the outlet port 34.

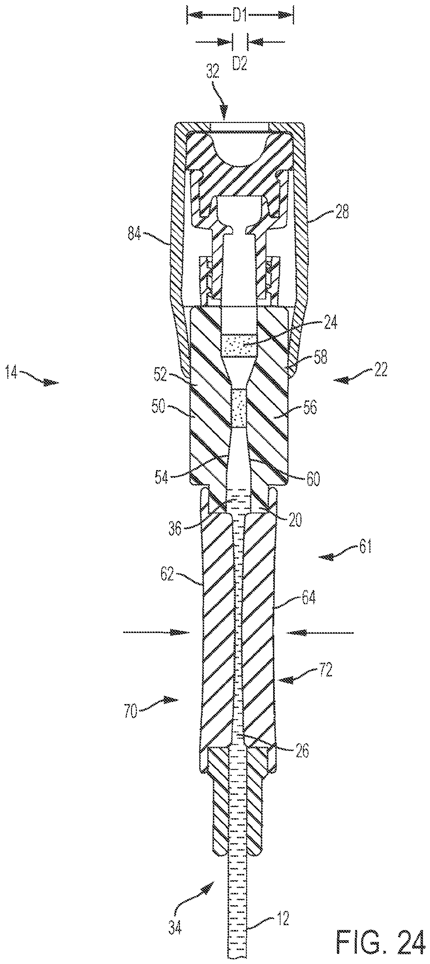

The internal portion of the mixing chamber 22 may have any suitable structure or form as long as it provides for the mixing of the blood sample 12 with an anticoagulant or another additive as the blood sample 12 passes through the passageway 36 of the collection module 14. Referring to Fig. 24, in one embodiment, the mixing chamber 22 includes a first curved wall 50 having a first inlet end 52 and a first exit end 54, and a second curved wall 56 having a second inlet end 58 and a second exit end 60. The first inlet end 52 is spaced a first distance D1 from the second inlet end 58 and the first exit end 54 is spaced a second distance D2 from the second exit end 60. In one embodiment, the second distance D2 is less than the first distance D1.

The mixing chamber 22 receives the sample 12 and the sample stabilizer 24 therein and effectuates distributed mixing of the sample stabilizer 24 within the sample 12. The mixing chamber 22 effectuates distributed mixing of the sample stabilizer 24 within the sample 12 and prevents a very high sample stabilizer concentration in any portion of the blood sample 12. This prevents underdosing of the sample stabilizer 24 in any portion of the blood sample 12. The mixing chamber 22 effectuates distributed mixing of the sample stabilizer 24 within the sample 12 so that an approximately equal amount and/or concentration of the sample stabilizer 24 is dissolved throughout the blood sample 12, e.g., an approximately equal amount and/or concentration of the sample stabilizer 24 is dissolved into the blood sample 12 from a front portion of the blood sample 12 to a rear portion of the blood sample 12.

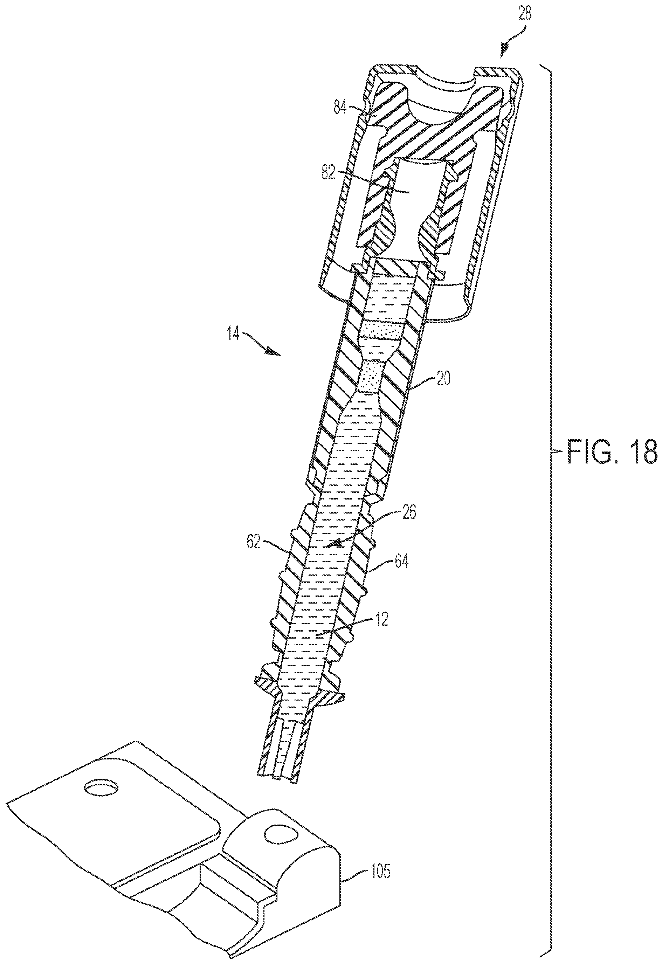

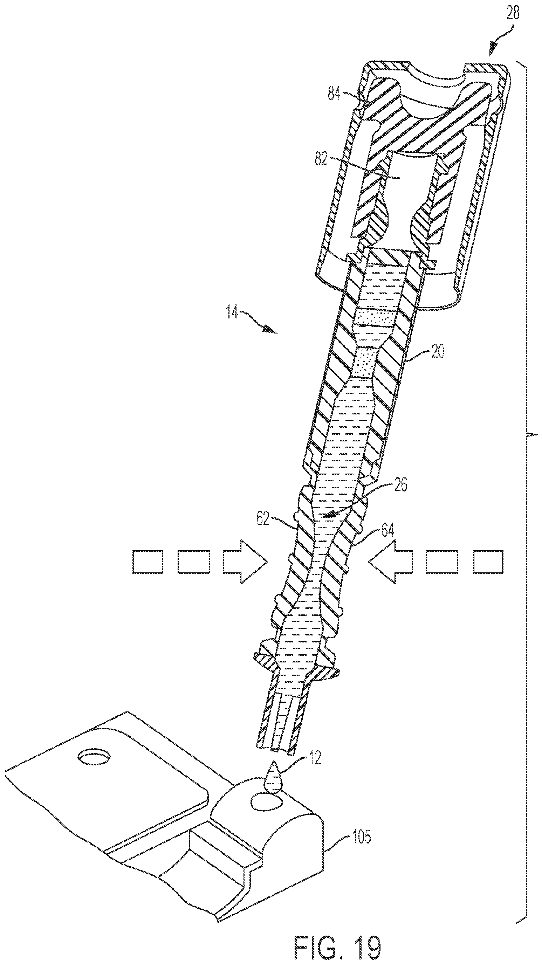

In one embodiment, the collection module 14 includes a collection chamber 26 that is disposed between the mixing chamber 22 and the outlet port 34. The collection chamber 26 includes an actuation portion 61. In one embodiment, the actuation portion 61 is transitionable between a first position (Figs. 18, 22, and 23) in which the sample 12 is containable within the collection chamber 26 and a second position (Figs. 19 and 24) in which a portion of the sample 12 is expelled from the collection chamber 26.

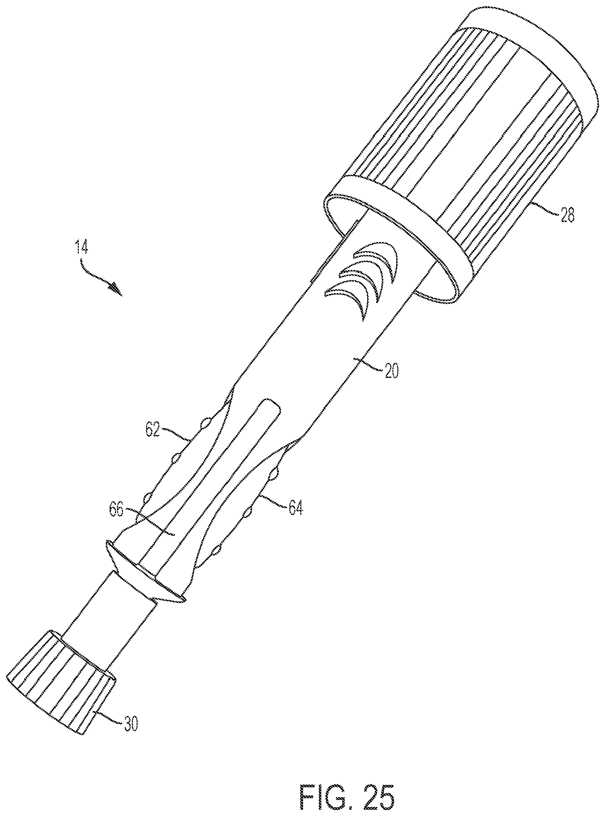

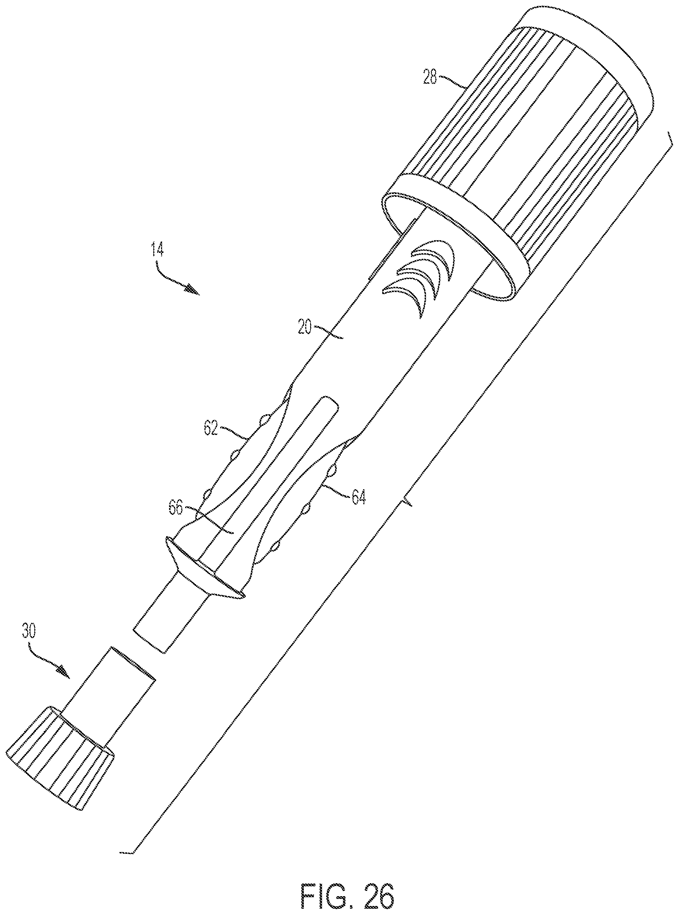

In one embodiment, the actuation portion 61 of the collection chamber 26 includes a first deformable portion 62, a second deformable portion 64, and a rigid wall portion 66 (Figs. 25 and 26) that is between the first deformable portion 62 and the second deformable portion 64. In one embodiment, the first deformable portion 62 is located on a first side 70 of the collection chamber 26 and the second deformable portion 64 is located on a second side 72 of the collection chamber 26. In one embodiment, the second side 72 of the collection chamber 26 is opposite from the first side 70 of the collection chamber 26.

In one embodiment, the first deformable portion 62 and the second deformable portion 64 are transitionable between an initial position (Figs. 18, 22, and 23) in which the sample 12 is contained within the collection chamber 26 and a deformed position (Figs. 19 and 24) in which a portion of the sample 12 is expelled from the collection chamber 26. The first deformable portion 62 and the second deformable portion 64 are simultaneously squeezed to transition from the initial position to the deformed position.

Advantageously, by having a first deformable portion 62 and a second deformable portion 64 that can be simultaneously squeezed, a collection module 14 of the present disclosure is able to dispense more sample 12 out of the collection chamber 26 and the outlet port 34. Furthermore, in one embodiment, by having a first deformable portion 62 on a first side 70 and a second deformable portion 64 on an opposite second side 72, a collection module 14 of the present disclosure has a symmetrical design and provides a smooth straight fluid path chamber that encourages fluid attachment flow characteristics. The smooth straight fluid path chamber of the collection module 14 is without significant geometric steps in diameter and the smooth fluid pathway inhibits the formation of air pockets or bubbles.

After passing through the mixing chamber 22, the stabilized sample is directed to the collection chamber 26. The collection chamber 26 may take any suitable shape and size to store a sufficient volume of blood necessary for the desired testing, for example, 500 µl or less. In one embodiment, the collection chamber 26 is defined by a portion of the housing 20 in combination with a first deformable portion 62, a second deformable portion 64, and a rigid wall portion 66.

The first deformable portion 62 and the second deformable portion 64 may be made of any material that is flexible, deformable, and capable of providing a fluid tight seal with the housing 20. In some embodiments, the first deformable portion 62 and the second deformable portion 64 may be made of natural or synthetic rubber, and other suitable elastomeric materials. The first deformable portion 62 and the second deformable portion 64 are secured to a portion of the housing 20 such that the first deformable portion 62 and the second deformable portion 64 are transitionable between an initial position (Figs. 18, 22, and 23) in which the sample 12 is contained within the collection chamber 26 and a deformed position (Figs. 19 and 24) in which a portion of the sample 12 is expelled from the collection chamber 26.

In another embodiment, the actuation portion 61 of the collection chamber 26 may comprise an activation member in accordance with an activation member described in U.S. Patent Application Serial No. 15/065,022, filed March 9, 2016, entitled "Biological Fluid Micro-Sample Management Device", the entire disclosure of which is hereby expressly incorporated herein by reference.

In other embodiments, the actuation portion 61 of the collection chamber 26 may comprise actuation portions in accordance with actuation portions and/or deformable portions described in U.S. Patent Application Serial No. 62/634,960, filed February 26, 2018, entitled "Biological Fluid Collection Device and Collection Module", the entire disclosure of which is hereby expressly incorporated herein by reference.

In one embodiment, the collection module 14 includes a cap 30 that is removably attachable to the outlet port 34 and that protectively covers the outlet port 34. In one embodiment, the cap 30 includes a venting plug 80 which allows air to pass therethrough and prevents the sample 12 from passing therethrough.

The construction of the cap 30 and venting plug 80 allows air to pass through the cap 30 while preventing the blood sample 12 from passing through the cap 30 and may include a hydrophobic filter. The venting plug 80 has selected air passing resistance that may be used to finely control the filling rate of the passageway 36 and/or the collection chamber 26 of the collection module 14. By varying the porosity of the plug, the velocity of the air flow out of the cap 30, and thus the velocity of the blood sample flow into the collection module 14, may be controlled.

In one embodiment, the collection module 14 includes a closure 28 that is engaged with the inlet port 32 of the collection module 14 to seal the passageway 36. The closure 28 protectively covers the inlet port 32. The closure 28 allows for introduction of a blood sample 12 into the passageway 36 of the housing 20 and may include a pierceable self-sealing stopper 82 with an outer shield 84 such as a Hemogard^{™} cap commercially available from Becton, Dickinson and Company.

The present disclosure provides a biological fluid collection system 10 that includes a power source 16 for a collection module 14 that receives a sample 12 and provides flow-through blood stabilization technology and a precise sample dispensing function for point-of-care and near patient testing applications. A power source of the present disclosure allows a user activated vacuum source.

In one embodiment, the power source 16 includes a spring loaded device for automatic drawing of a blood sample 12 within the collection module 14. A spring loaded power source utilizes a user activated, spring powered piston to generate a vacuum on a distal end of a collection module 14. In such an embodiment, by controlling the stiffness of and travel length of the spring, a predictable vacuum can be applied to a fluid path of the collection module 14 to generate a given flow rate of blood as it fills the collection module 14. Predictable flow rates are important for the mixing structure.

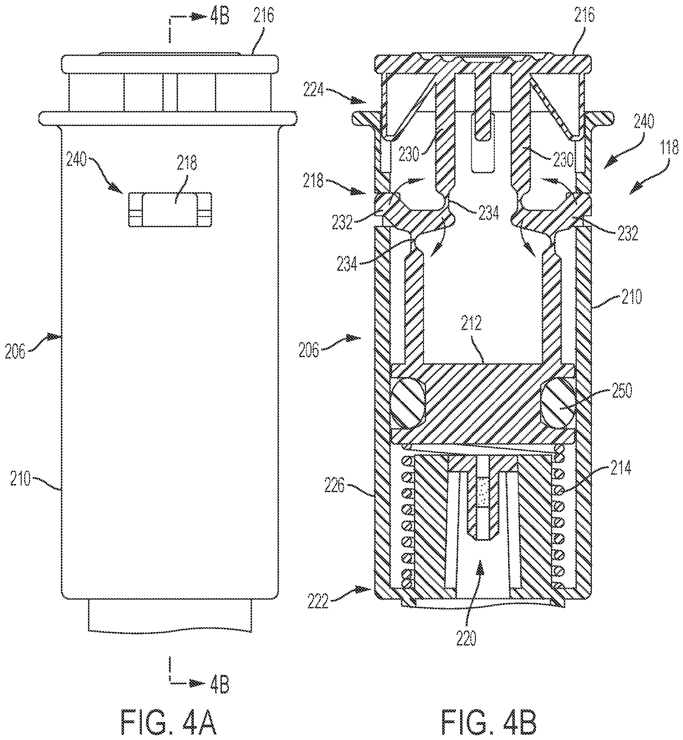

Referring to Figs. 1-3, in one exemplary embodiment, a power source 16 is removably connectable with a collection module 14 and the power source 16 creates a vacuum that draws a sample 12 within the collection chamber 26. In one embodiment, the power source 16 includes a barrel 110, a piston 112, a spring 114, an activation button 116, and a lock 118 (exemplary embodiments shown in Figs. 4A-8E). In one embodiment, the piston 112 includes an O-ring 150 that provides stiction with the interior surface of a sidewall 126 of the barrel 110.

The barrel 110 is in communication with the collection chamber 26 of the collection module 14. The barrel 110 defines an interior 120 and includes a first end 122, a second end 124, and a sidewall 126 therebetween. The barrel 110 is removably connectable with a portion of the collection module 14. For example, in one embodiment, the barrel 110 is removably connectable with the cap 30 of the collection module 14 such that a vacuum created by the power source 16 is able to draw a sample 12 within the collection chamber 26 of the collection module 14. As discussed above, the cap 30 includes a venting plug 80 which allows air to pass therethrough and prevents the sample 12 from passing therethrough. In this manner, the vacuum created within the barrel 110 of the power source 16 is in communication with the collection chamber 26 of the collection module 14 such that a vacuum created by the power source 16 is able to draw a sample 12 within the collection chamber 26 of the collection module 14.

The piston 112 is slidably disposed within the interior 120 of the barrel 110. The piston 112 is sized relative to the interior 120 of the barrel 110 to provide sealing engagement with the sidewall 126 of the barrel 110. The piston 112 is transitionable between a first piston position (Fig. 1), in which the piston 112 is a first distance from the first end 122 of the barrel 110, and a second piston position (Fig. 2), in which the piston 112 is a second distance from the first end 122 of the barrel 110, the second distance greater than the first distance.

Referring to Figs. 1-3, the spring 114 is disposed between the first end 122 of the barrel 110 and the piston 112. In one embodiment, the activation button 116 is disposed on a portion of the barrel 110.

The power source 16 also includes a lock 118 that is in communication with the spring 114 and the activation button 116. The lock 118 is transitionable between a locked position, in which the lock 118 locks the piston 112 in the first piston position (Fig. 1) and maintains the spring 114 in a compressed position, and an unlocked position, in which the piston 112 is unlocked and the spring 114 is permitted to drive the piston 112 to the second piston position (Fig. 2) thereby creating a vacuum that pulls the sample 12 within the collection chamber 26 of the collection module 14. In one embodiment, actuation of the activation button 116 moves the lock 118 to the unlocked position.

Exemplary embodiments of a lock 118 of a power source of the present disclosure will now be discussed. Referring to Figs. 4A-6C, in an exemplary embodiment, a power source 206 is removably connectable with a collection module 14 and the power source 206 creates a vacuum that draws a sample 12 within the collection chamber 26. In one embodiment, the power source 206 includes a barrel 210, a piston 212, a spring 214, an activation button 216, and a lock 218.

The barrel 210 is in communication with the collection chamber 26 of the collection module 14. The barrel 210 defines an interior 220 and includes a first end 222, a second end 224, and a sidewall 226 therebetween. The barrel 210 is removably connectable with a portion of the collection module 14. For example, the barrel 210 is removably connectable with the cap 30 of the collection module 14 such that a vacuum created by the power source 206 is able to draw a sample 12 within the collection chamber 26 of the collection module 14. As discussed above, the cap 30 includes a venting plug 80 which allows air to pass therethrough and prevents the sample 12 from passing therethrough. In this manner, the vacuum created within the barrel 210 of the power source 206 is in communication with the collection chamber 26 of the collection module 14 such that a vacuum created by the power source 206 is able to draw a sample 12 within the collection chamber 26 of the collection module 14.

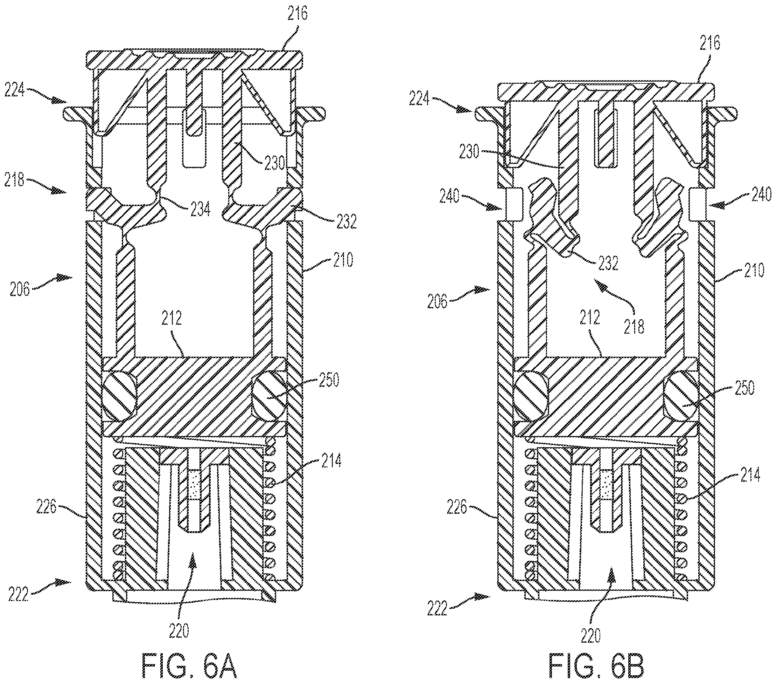

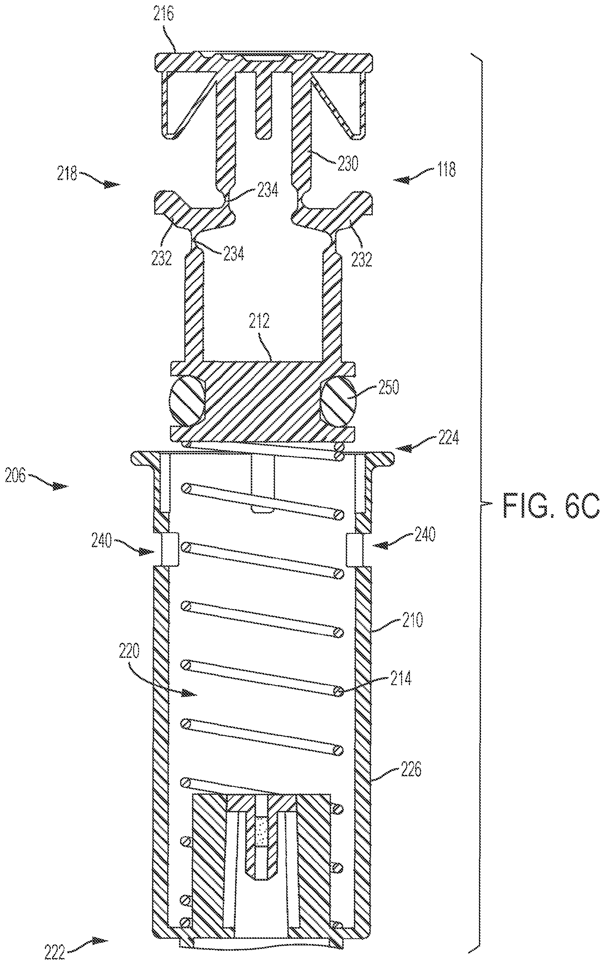

The piston 212 is slidably disposed within the interior 220 of the barrel 210. The piston 212 is sized relative to the interior 220 of the barrel 210 to provide sealing engagement with the sidewall 226 of the barrel 210. The piston 212 is transitionable between a first piston position (Fig. 6A), in which the piston 212 is a first distance from the first end 222 of the barrel 210, and a second piston position (Fig. 6C), in which the piston 212 is a second distance from the first end 222 of the barrel 210, the second distance greater than the first distance. In one embodiment, the piston 212 includes an O-ring 250 that provides stiction with the interior surface of the sidewall 226 of the barrel 210.

Referring to Figs. 6A-6C, the spring 214 is disposed between the first end 222 of the barrel 210 and the piston 212. The spring 214 is maintained in a pre-loaded position with the lock 218 in the locked position, in which the lock 218 locks the piston 212 in the first piston position and maintains the spring 214 in a compressed position. In one embodiment, the activation button 216 is disposed on a portion of the barrel 210.

The power source 206 also includes a lock 218 that is in communication with the spring 214 and the activation button 216. The lock 218 is transitionable between a locked position, in which the lock 218 locks the piston 212 in the first piston position (Fig. 6A) and maintains the spring 214 in a compressed position, and an unlocked position, in which the piston 212 is unlocked and the spring 214 is permitted to drive the piston 212 to the second piston position (Fig. 6C) thereby creating a vacuum that pulls the sample 12 within the collection chamber 26 of the collection module 14. In one embodiment, actuation of the activation button 216 moves the lock 218 to the unlocked position.

Referring to Figs. 4A-6C, in one embodiment, the lock 218 includes the activation button 216, button longitudinal portions 230, rotatable locking clips 232, and bendable portions 234. The barrel 210 includes a pair of sidewall apertures 240 that respectively receive rotatable locking clips 232 in the locked position (Fig. 6A).

Referring to Fig. 6A, with the lock 218 in the locked position, the rotatable locking clips 232 are locked within the respective sidewall apertures 240 of the barrel 210. In the locked position, the lock 218 locks the piston 212 in the first piston position and maintains the spring 214 in a compressed position.

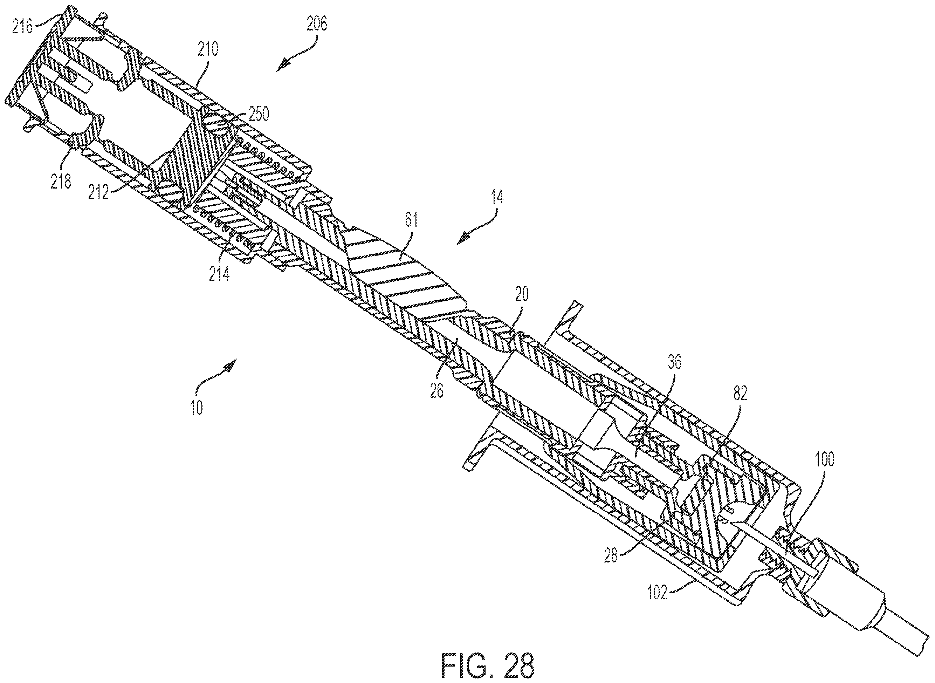

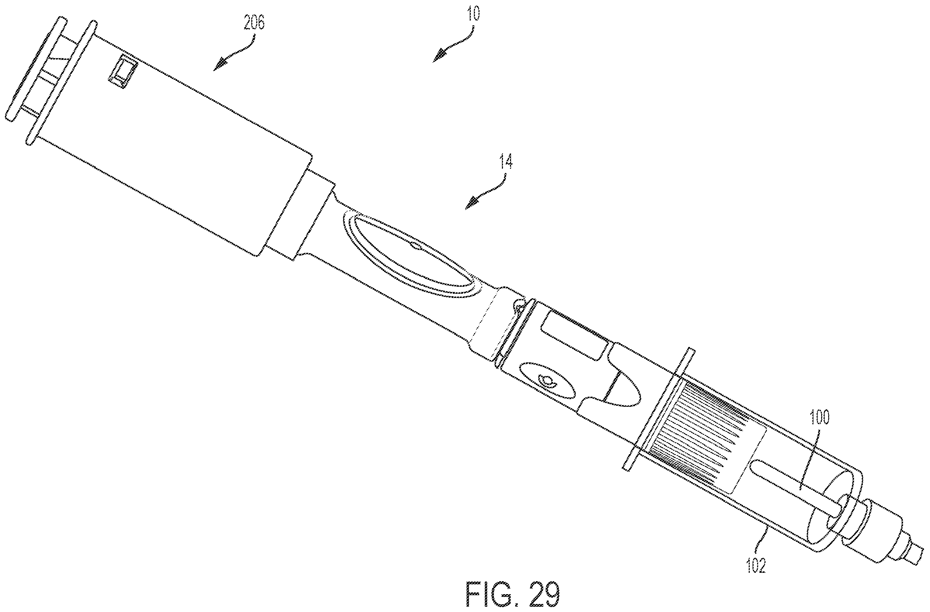

Referring to Figs. 4A-6C and 27-29, use of a biological fluid collection system 10 of the present disclosure having a collection module 14 and a power source 206 will now be described. In use, a needle cannula 100 (Figs. 28 and 29) is inserted into the passageway 36 of the housing 20 of the collection module 14 through the inlet port 32, such as through the pierceable self-sealing stopper 82 of closure 28. Referring to Figs. 4A-6C and 27-29, the biological fluid collection system 10 including the collection module 14 and the power source 206 may be inserted into a conventional tube holder 102 having a cannula 100 through which biological fluid, such as a blood sample 12, is passed.

When a user desires to pull a blood sample 12 into the collection module 14 from the conventional tube holder 102 by the draw of a vacuum created within the power source 206, the user actuates, i.e., pushes down, the activation button 216 which moves the lock 218 to the unlocked position (Figs. 6B and 6C). Referring to Fig. 6B, pushing down on the activation button 216 forces the button longitudinal portions 230 to move downward thereby rotating the locking clips 232 inwardly and out of engagement with the sidewall apertures 240 of the barrel 210. In this manner, the locking clips 232 of the lock 218 are rotated into the unlocked position (Figs. 6B and 6C). In one embodiment, the locking clips 232 rotate about the bendable portions 234. In one embodiment, as the activation button 216 is pressed, e.g., pushed down, a stiction is broken between an O-ring 250 and the interior surface of the sidewall 226 of the barrel 210.

With the lock 218 in the unlocked position (Figs. 6B and 6C), the piston 212 is unlocked and the spring 214 is permitted to drive the piston 212 to the second piston position (Fig. 6C) thereby creating a vacuum within the barrel 210 that pulls a blood sample 12 within the collection chamber 26 of the collection module 14 from the conventional tube holder 102.

Advantageously, a collection module and a power source of the present disclosure can be engaged with many different sources through which biological fluid, such as a blood sample 12, is passed. For example, in some embodiments, a collection module and a power source of the present disclosure can be engaged with a conventional tube holder 102 as described above. In other embodiments, a user activated power source of the present disclosure enables the user to connect directly to a Luer-line, e.g., IV Catheter, wingset, PICC, or similar device. In other embodiments, if the collection module and the power source are used with a HemoLuer, a user may connect the collection module and the power source to either a Luer (by removing the HemoLuer) or a conventional tube holder (using the HemoLuer as an interface). Advantageously, the system of the present disclosure also allows for direct Luer access without the use of an LLAD (Luer Line Access Device) or any other holder.

The blood sample 12 is pulled into the passageway 36 of the housing 20 of the collection module 14 from the conventional tube holder 102 by the draw of the vacuum created in the barrel 210. In one embodiment, the blood sample 12 fills the entire passageway 36 such that, as the blood sample 12 enters the collection module 14, the blood sample 12 passes through the open cell foam, e.g., the material 40, and is exposed to the anticoagulant powder 44 available throughout the internal pore 42 structure of the open cell foam. In this manner, the sample 12 dissolves and mixes with the dry anticoagulant powder 44 while passing through the material 40 or open cell foam. Next, the mixing chamber 22 receives the sample 12 and the sample stabilizer 24 therein and effectuates distributed mixing of the sample stabilizer 24 within the sample 12. After passing through the mixing chamber 22, the stabilized sample is directed to the collection chamber 26. The collection chamber 26 may take any suitable shape and size to store a sufficient volume of blood necessary for the desired testing, for example, 500 µl or less. In one embodiment, the cap 30 stops the collection of the blood sample 12 when the passageway 36, the mixing chamber 22, and the collection chamber 26 of the collection module 14 have been fully filled. The venting plug 80 of the cap 30 allows air to pass through the cap 30 while preventing the blood sample 12 from passing through the cap 30 into the barrel 210 of the power source 206.

In one embodiment, once sample collection is complete, the power source 206 and the collection module 14 are separated from the tube holder 102 (Fig. 29), and then the power source 206 is separated from the collection module 14 (Fig. 25).

Once the collection module 14 is separated from the power source 206, the cap 30 may then be removed from the collection module 14 (Fig. 26) exposing the outlet port 34 of the housing 20 of the collection module 14. Removal may be accomplished by the user grasping an exterior portion of the cap 30 and pulling the cap 30 from the housing 20. The blood sample 12 is held within the passageway 36 of the housing 20, e.g., the collection chamber 26, by capillary action after removal of the cap 30.

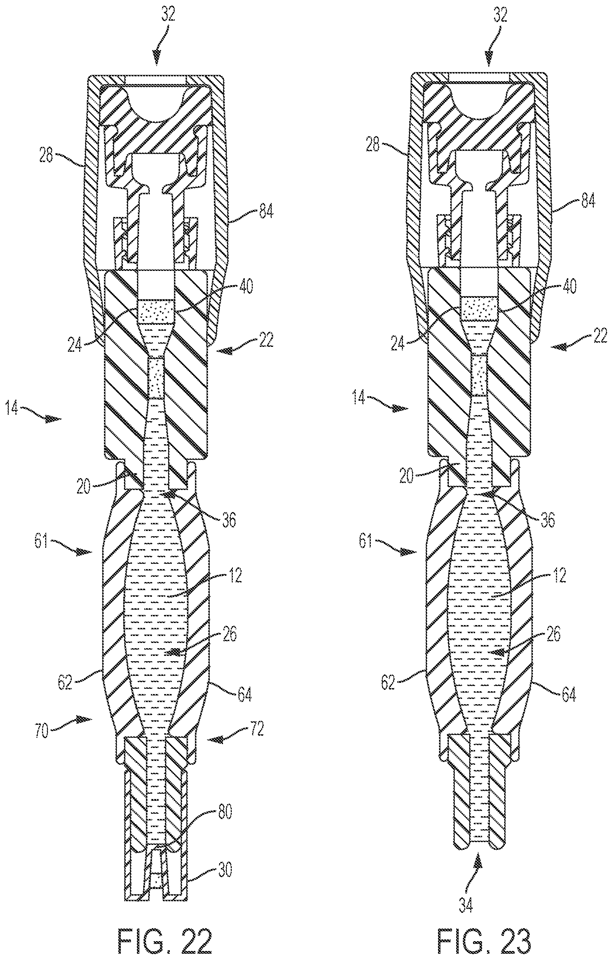

The blood sample 12 may then be dispensed from the collection module 14 by activation of the actuation portion 61. In one embodiment, the actuation portion 61 includes a first deformable portion 62 and a second deformable portion 64. For example, the first deformable portion 62 and the second deformable portion 64 are transitionable between an initial position (Figs. 18 and 23) in which the sample 12 is contained within the collection chamber 26 and a deformed position (Figs. 19 and 24) in which a portion of the sample 12 is expelled from the collection chamber 26 and the outlet port 34. The first deformable portion 62 and the second deformable portion 64 are simultaneously squeezed to transition from the initial position (Figs. 18 and 23) to the deformed position (Figs. 19 and 24). In this manner, the blood sample 12 may be transferred to a device intended to analyze the sample, e.g., such as a point-of-care testing device 105 (Figs. 18 and 19), a cartridge tester, or a near patient testing device, while minimizing the exposure of the medical practitioner to the blood sample.

Advantageously, by having a first deformable portion 62 and a second deformable portion 64 that can be simultaneously squeezed, a collection module 14 of the present disclosure is able to dispense more sample 12 out of the collection chamber 26 and the outlet port 34. Furthermore, in one embodiment, by having a first deformable portion 62 on a first side 70 and a second deformable portion 64 on an opposite second side 72, a collection module 14 of the present disclosure has a symmetrical design and provides a smooth straight fluid path chamber that encourages fluid attachment flow characteristics.

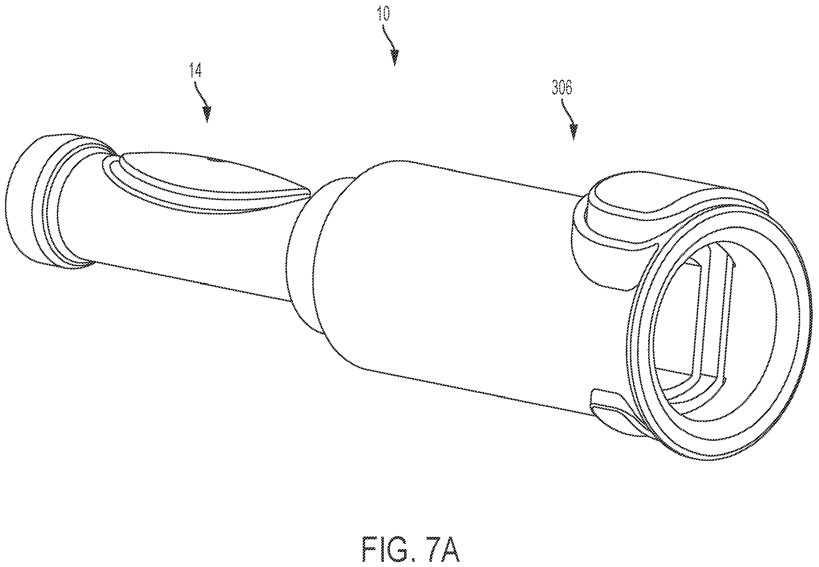

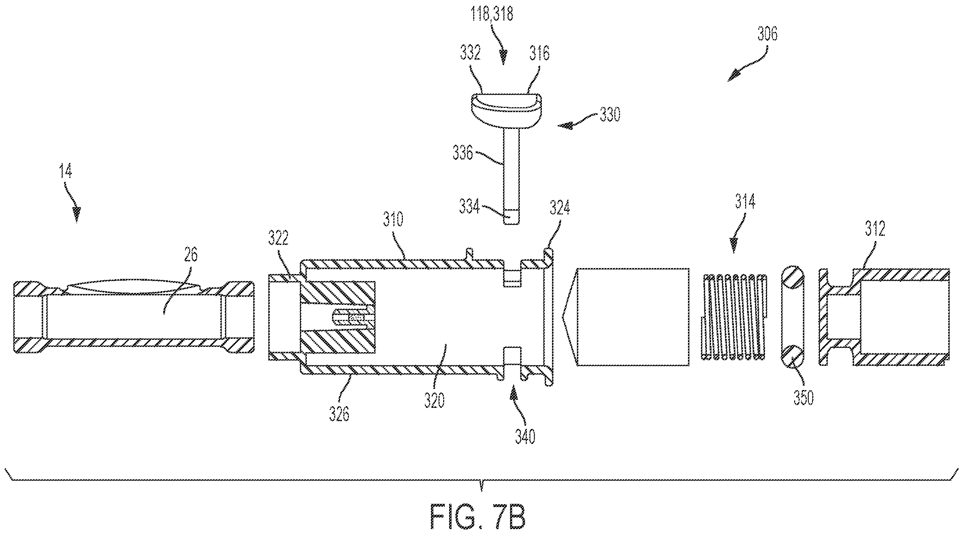

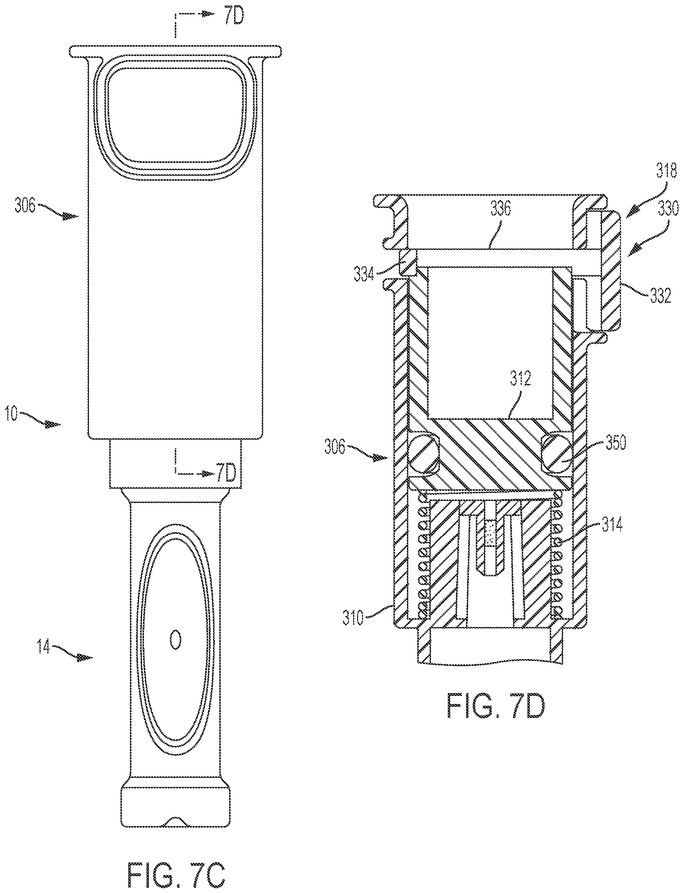

Another exemplary embodiment of a lock 118 of a power source will now be discussed. Referring to Figs. 7A-8E, in an exemplary embodiment, a power source 306 is removably connectable with a collection module 14 and the power source 306 creates a vacuum that draws a sample 12 within the collection chamber 26. In one embodiment, the power source 306 includes a barrel 310, a piston 312, a spring 314, an activation button 316, and a lock 318.

The barrel 310 is in communication with the collection chamber 26 of the collection module 14. The barrel 310 defines an interior 320 and includes a first end 322, a second end 324, and a sidewall 326 therebetween. The barrel 310 is removably connectable with a portion of the collection module 14. For example, the barrel 310 is removably connectable with the cap 30 of the collection module 14 such that a vacuum created by the power source 306 is able to draw a sample 12 within the collection chamber 26 of the collection module 14. As discussed above, the cap 30 includes a venting plug 80 which allows air to pass therethrough and prevents the sample 12 from passing therethrough. In this manner, the vacuum created within the barrel 310 of the power source 306 is in communication with the collection chamber 26 of the collection module 14 such that a vacuum created by the power source 306 is able to draw a sample 12 within the collection chamber 26 of the collection module 14.

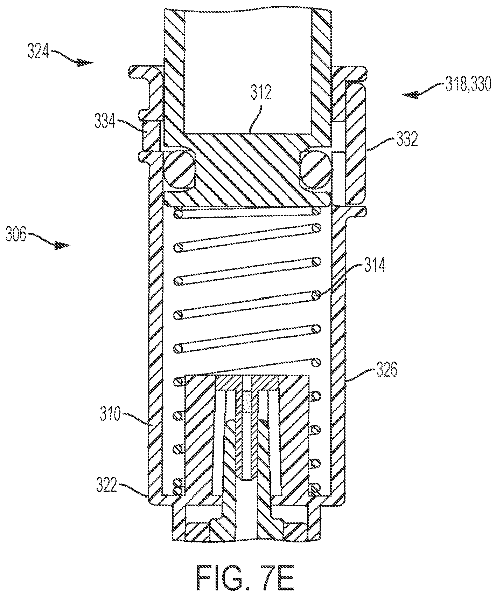

The piston 312 is slidably disposed within the interior 320 of the barrel 310. The piston 312 is sized relative to the interior 320 of the barrel 310 to provide sealing engagement with the sidewall 326 of the barrel 310. The piston 312 is transitionable between a first piston position (Fig. 7D), in which the piston 312 is a first distance from the first end 322 of the barrel 310, and a second piston position (Fig. 7E), in which the piston 312 is a second distance from the first end 322 of the barrel 310, the second distance greater than the first distance. In one embodiment, the piston 312 includes an O-ring 350 that provides stiction with the interior surface of the sidewall 326 of the barrel 310.

Referring to Figs. 7D-7E, the spring 314 is disposed between the first end 322 of the barrel 310 and the piston 312. The spring 314 is maintained in a pre-loaded position with the lock 318 in the locked position, in which the lock 318 locks the piston 312 in the first piston position and maintains the spring 314 in a compressed position. In one embodiment, the activation button 316 is disposed on a portion of the barrel 310.

The power source 306 also includes a lock 318 that is in communication with the spring 314 and the activation button 316. The lock 318 is transitionable between a locked position, in which the lock 318 locks the piston 312 in the first piston position (Fig. 7D) and maintains the spring 314 in a compressed position, and an unlocked position, in which the piston 312 is unlocked and the spring 314 is permitted to drive the piston 312 to the second piston position (Fig. 7E) thereby creating a vacuum that pulls the sample 12 within the collection chamber 26 of the collection module 14. In one embodiment, actuation of the activation button 316 moves the lock 318 to the unlocked position.

Referring to Figs. 7A-8E, in one embodiment, the lock 318 includes a cinch ring 330 including a button portion 332, a barrier portion 334, and a ring portion 336. The barrel 310 includes a sidewall aperture 340 that receives the cinch ring 330. In one embodiment, the activation button 316 is the button portion 332.

Referring to Fig. 7D, with the lock 318 in the locked position, the barrier portion 334 extends into the barrel 310 and contacts a portion of the piston 312 to lock the piston 312 in the first piston position and maintain the spring 314 in a compressed position. In this manner, the barrier portion 334 of the cinch ring 330 acts as a physical barrier to prevent piston from movement within the barrel 310 and to lock the piston 312 in the first piston position and maintain the spring 314 in a compressed position.

Referring to Figs. 7D-7E, use of a biological fluid collection system 10 of the present disclosure having a collection module 14 and a power source 306 will now be described. Use of the embodiment illustrated in Figs. 7A-8E involves similar steps of use as the embodiment illustrated in Figs. 4A-6C, as described in detail above. For the sake of brevity, these similar steps of using a biological fluid collection system 10 of the present disclosure having a collection module 14 and a power source 306 will not all be discussed in conjunction with the embodiment illustrated in Figs. 7A-8E.

In use, as described above, a needle cannula 100 (Figs. 28 and 29) is inserted into the passageway 36 of the housing 20 of the collection module 14 through the inlet port 32, such as through the pierceable self-sealing stopper 82 of closure 28. As described above, in one embodiment, the biological fluid collection system 10 including the collection module 14 and the power source 306 may be inserted into a conventional tube holder 102 having a cannula 100 through which biological fluid, such as a blood sample 12, is passed.

When a user desires to pull a blood sample 12 into the collection module 14 from the conventional tube holder 102 by the draw of a vacuum created within the power source 306, the user actuates, i.e., pushes in, the button portion 332 which moves the lock 318 to the unlocked position (Fig. 7E). Referring to Fig. 7E, pushing the button portion 332 in forces the barrier portion 334 to move outward thereby disengaging from contact with the piston 312. In this manner, the lock 318 is moved to the unlocked position. In one embodiment, as the button portion 332 is pressed, e.g., pushed in, a stiction is broken between an O-ring 350 and the interior surface of the sidewall 326 of the barrel 310.

With the lock 318 in the unlocked position (Fig. 7E), the piston 312 is unlocked and the spring 314 is permitted to drive the piston 312 to the second piston position (Fig. 7E) thereby creating a vacuum within the barrel 310 that pulls a blood sample 12 within the collection chamber 26 of the collection module 14 from the conventional tube holder 102.

As described above, once sample collection is complete, the power source 306 and the collection module 14 are separated from the tube holder 102 (Fig. 29), and then the power source 306 is separated from the collection module 14 (Fig. 25).

Once the collection module 14 is separated from the power source 306, the cap 30 may then be removed from the collection module 14 (Fig. 26) exposing the outlet port 34 of the housing 20 of the collection module 14. Removal may be accomplished by the user grasping an exterior portion of the cap 30 and pulling the cap 30 from the housing 20. The blood sample 12 is held within the passageway 36 of the housing 20, e.g., the collection chamber 26, by capillary action after removal of the cap 30.

As described above, the blood sample 12 may then be dispensed from the collection module 14 by activation of the actuation portion 61 as shown in Figs. 18 and 19.

The present disclosure provides a biological fluid collection system 10 that includes a power source 16 for a collection module 14 that receives a sample 12 and provides flow-through blood stabilization technology and a precise sample dispensing function for point-of-care and near patient testing applications. A power source of the present disclosure allows a user activated vacuum source.

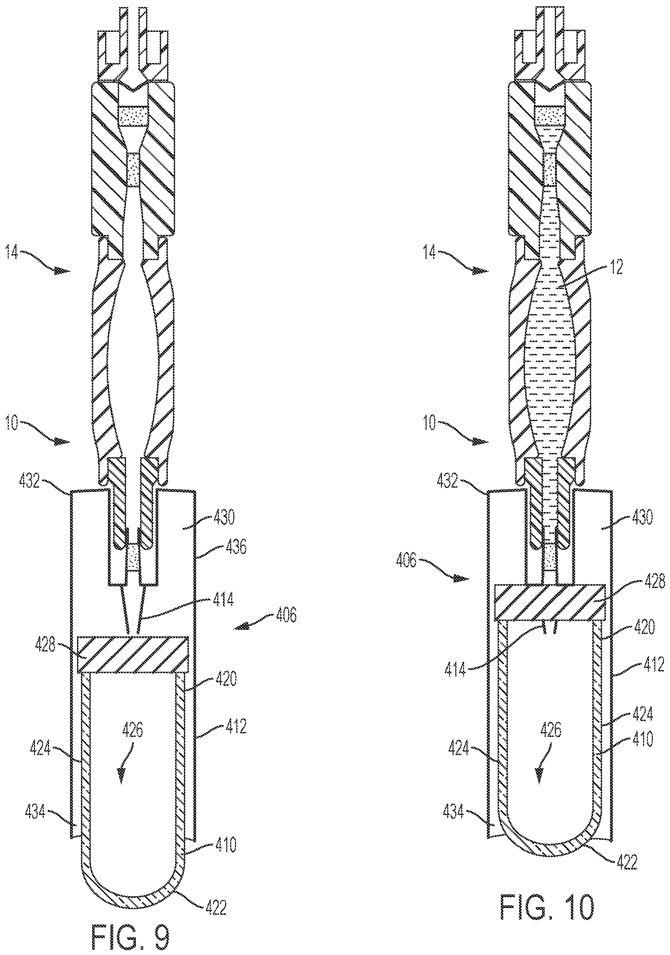

Referring to Figs. 9-14, the power source 406 includes an evacuated tube and tube holder device for automatic drawing of a blood sample 12 within the collection module 14.

Referring to Figs. 9-11, in one exemplary embodiment, a power source 406 is removably connectable with a collection module 14 and the power source 406 has a vacuum that draws a sample 12 within the collection chamber 26. In one embodiment, the power source 406 includes an evacuated tube 410, a tube holder 412, and a spike 414.

The evacuated tube 410 includes a first tube end 420, a second tube end 422, and a sidewall 424 extending therebetween and defining a tube interior 426. The evacuated tube contains the vacuum. The evacuated tube 410 includes a closure 428 sealing the first tube end 420.

The tube holder 412 is removably connectable with a portion of the collection module 14. In one embodiment, the tube holder 412 defines an interior 430 and includes a first end 432, a second end 434, and a tube holder sidewall 436 therebetween.

In one embodiment, the spike 414 includes a first spike end 440 and a second spike end 442. The spike 414 is removably connectable with a portion of the collection module 14 and with a portion of the power source 406. The spike 414 is able to be placed in communication with the collection chamber 26 of the collection module 14.

In one embodiment, the evacuated tube 410 is movably disposed within the interior 430 of the tube holder 412 between a first tube position (Fig. 9), in which the evacuated tube 410 is disengaged from the spike 414, and a second tube position (Fig. 10), in which the closure 428 of the evacuated tube 410 is pierced by the spike 414.

In one embodiment, with the evacuated tube 410 in the first tube position (Fig. 9), a portion of the second tube end 422 is exposed from the second end 434 of the tube holder 412 and the second tube end 422 can be pushed to move the evacuated tube 410 to the second tube position (Fig. 10). Referring to Figs. 9-11, in one embodiment, the second tube end 422 comprises an arcuate surface.

Referring to Figs. 9-11, use of a biological fluid collection system 10 of the present disclosure having a collection module 14 and a power source 406 will now be described. Use of the embodiment illustrated in Figs. 9-11 involves similar steps of use as the embodiment illustrated in Figs. 4A-6C, as described in detail above. For the sake of brevity, these similar steps of using a biological fluid collection system 10 of the present disclosure having a collection module 14 and a power source 406 will not all be discussed in conjunction with the embodiment illustrated in Figs. 9-11.

In use, as described above, a needle cannula 100 (Figs. 28 and 29) is inserted into the passageway 36 of the housing 20 of the collection module 14 through the inlet port 32, such as through the pierceable self-sealing stopper 82 of closure 28. As described above, in one embodiment, the biological fluid collection system 10 including the collection module 14 and the power source 406 may be inserted into a conventional tube holder 102 having a cannula 100 through which biological fluid, such as a blood sample 12, is passed.

When a user desires to pull a blood sample 12 into the collection module 14 from the conventional tube holder 102 by the draw of a vacuum within the power source 406, the user actuates, i.e., pushes down, the second tube end 422 of the evacuated tube 410 which moves the evacuated tube 410 to the second tube position (Fig. 10). Referring to Fig. 10, pushing down on the evacuated tube 410 forces the spike 414 to pierce the closure 428 of the evacuated tube 410. In this manner, the vacuum contained within the evacuated tube 410 is in communication with the collection chamber 26 of the collection module 14 via the spike 414 and the vacuum of the evacuated tube 410 draws the sample 12 within the collection chamber 26 of the collection module 14.

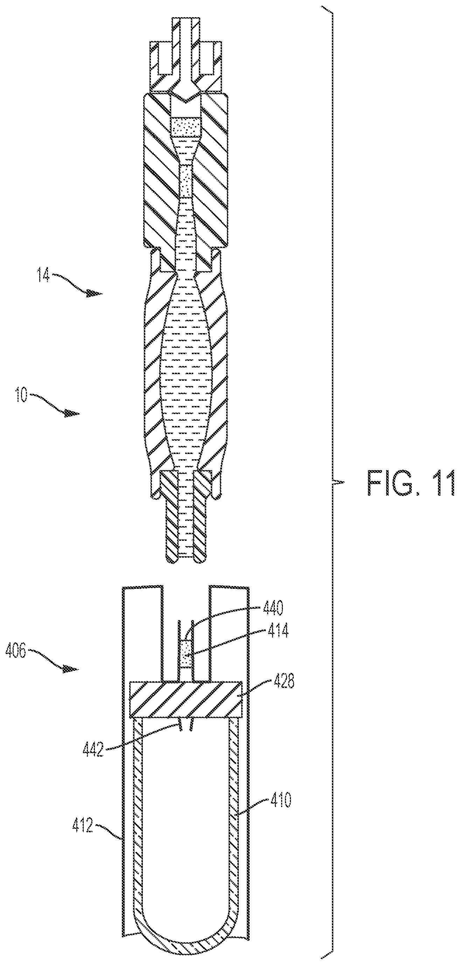

As described above, once sample collection is complete, the power source 406 and the collection module 14 are separated from the tube holder 102 (Fig. 29), and then the power source 406 is separated from the collection module 14 (Fig. 11). Referring to Fig. 11, in one embodiment, with the collection module 14 separated from the power source 406, the spike 414 remains in the evacuated tube 410.

Once the collection module 14 is separated from the power source 406, the cap 30 may then be removed from the collection module 14 (Fig. 26) exposing the outlet port 34 of the housing 20 of the collection module 14. Removal may be accomplished by the user grasping an exterior portion of the cap 30 and pulling the cap 30 from the housing 20. The blood sample 12 is held within the passageway 36 of the housing 20, e.g., the collection chamber 26, by capillary action after removal of the cap 30.

As described above, the blood sample 12 may then be dispensed from the collection module 14 by activation of the actuation portion 61 as shown in Figs. 18 and 19.

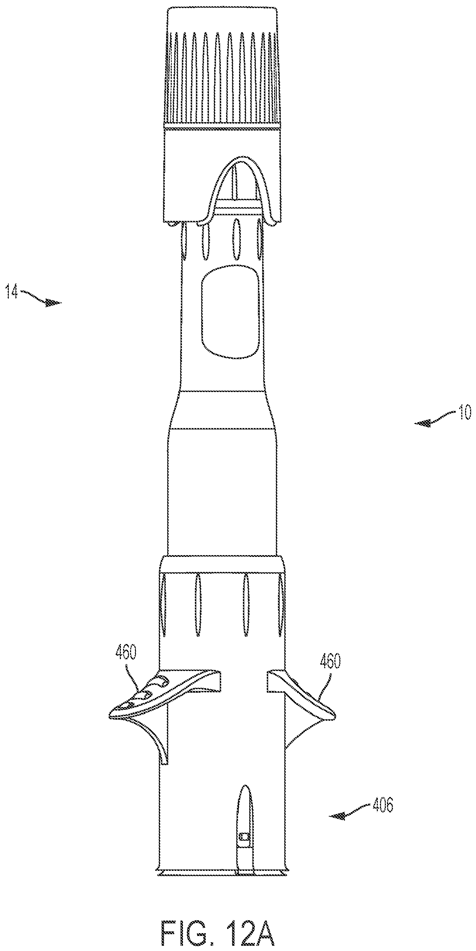

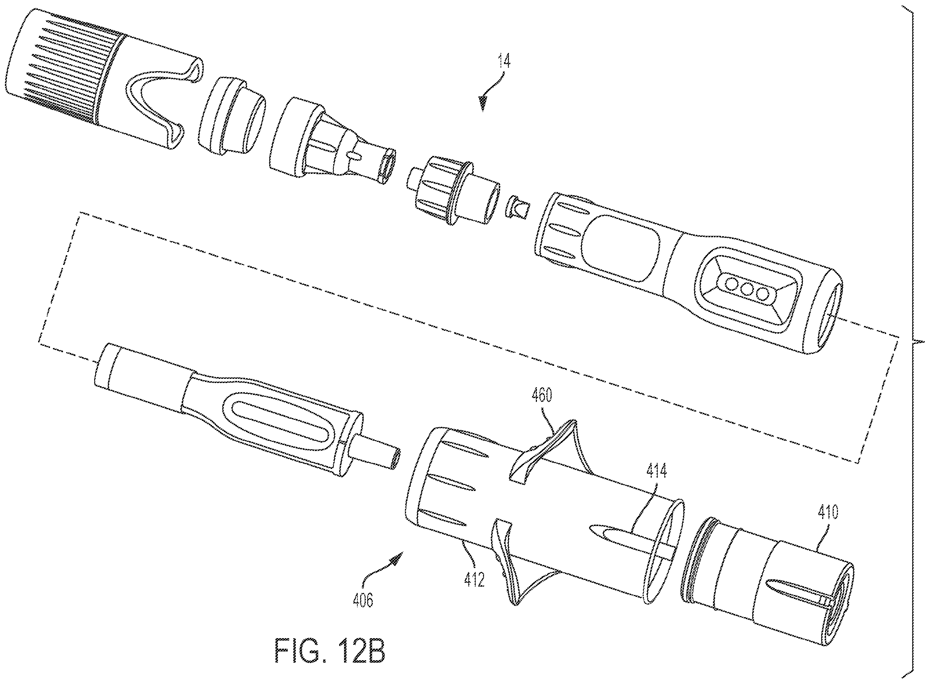

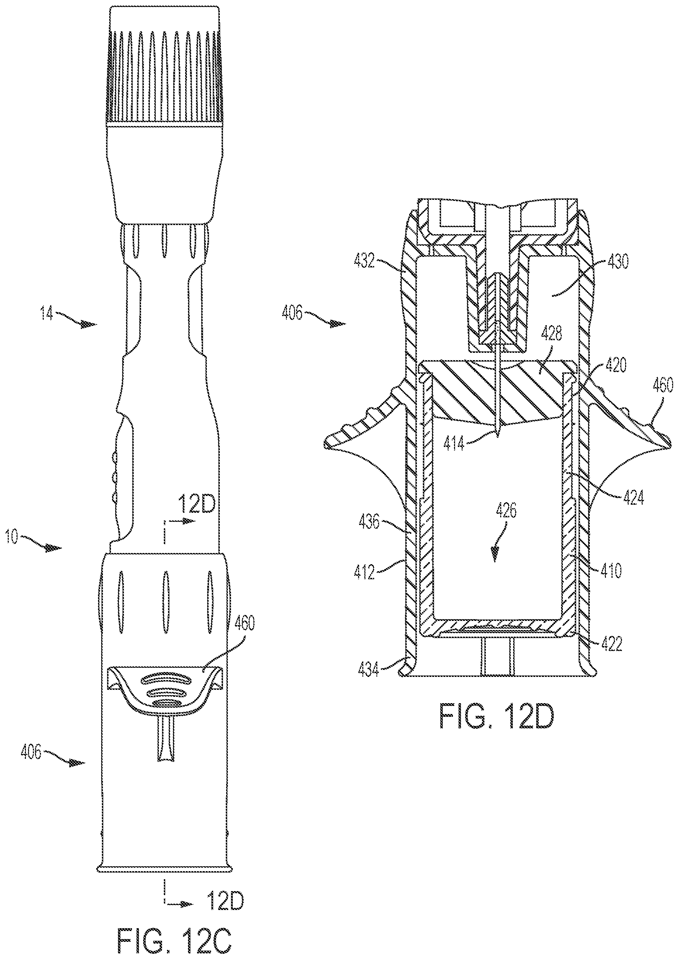

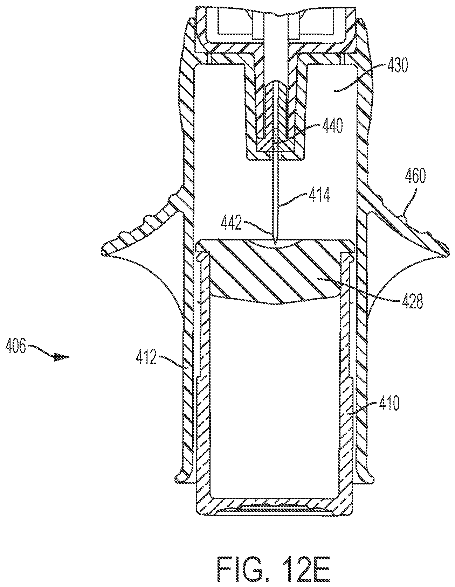

Figs. 12A-14C illustrate other exemplary embodiments of a biological fluid collection system 10 including a power source 406 having an evacuated tube 410 and tube holder 412 device for automatic drawing of a blood sample 12 within the collection module 14. The embodiments illustrated in Figs. 12A-14C include similar components to the embodiment illustrated in Figs. 9-11. For the sake of brevity, these similar components and the similar steps of using these devices will not all be discussed in conjunction with the embodiments illustrated in Figs. 12A-14C.

Referring to Figs. 12A-14C, in one embodiment, the tube holder 412 of the power source 406 includes finger flange portions 460 that facilitate the handling and use of the power source 406.

The present disclosure provides a biological fluid collection system 10 that includes a power source 16 for a collection module 14 that receives a sample 12 and provides flow-through blood stabilization technology and a precise sample dispensing function for point-of-care and near patient testing applications. A power source of the present disclosure allows a user activated vacuum source.

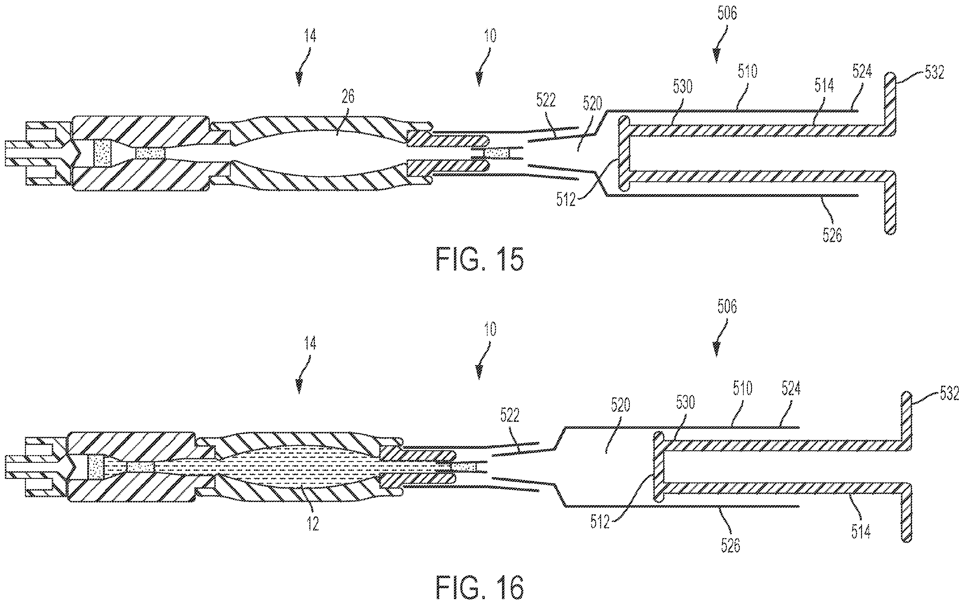

Referring to Figs. 15-17, the power source 506 includes a syringe assembly for automatic drawing of a blood sample 12 within the collection module 14.

Referring to Figs. 15-17, in one exemplary embodiment, a power source 506 is removably connectable with a collection module 14 and the power source 506 creates a vacuum that draws a sample 12 within the collection chamber 26. In one embodiment, the power source 506 includes a barrel 510, a stopper 512, and a plunger 514. In one embodiment, the barrel 510, the stopper 512, and the plunger 514 are part of a syringe assembly.

The barrel 510 is in communication with the collection chamber 26 of the collection module 14. The barrel 510 defines an interior 520 and includes a first end 522, a second end 524, and a sidewall 526 therebetween. The barrel 510 is removably connectable with a portion of the collection module 14. For example, the barrel 510 is removably connectable with the cap 30 of the collection module 14 such that a vacuum created by the power source 506 is able to draw a sample 12 within the collection chamber 26 of the collection module 14. As discussed above, the cap 30 includes a venting plug 80 which allows air to pass therethrough and prevents the sample 12 from passing therethrough. In this manner, the vacuum created within the barrel 510 of the power source 506 is in communication with the collection chamber 26 of the collection module 14 such that a vacuum created by the power source 506 is able to draw a sample 12 within the collection chamber 26 of the collection module 14.

The stopper 512 is slidably disposed within the interior 520 of the barrel 510. The stopper 512 is sized relative to the interior 520 of the barrel 510 to provide sealing engagement with the sidewall 526 of the barrel 510. The stopper 512 is transitionable between a first stopper position (Fig. 15), in which the stopper 512 is a first distance from the first end 522 of the barrel 510, and a second stopper position (Fig. 16), in which the stopper 512 is a second distance from the first end 522 of the barrel 510, the second distance greater than the first distance.

The plunger 514 includes a first plunger end 530 and a second plunger end 532. In one embodiment, a portion of the first plunger end 530 is engaged with the stopper 512, wherein movement of the plunger 514 away from the first end 522 of the barrel 510 moves the stopper 512 to the second stopper position (Fig. 16) thereby creating a vacuum that pulls the sample 12 within the collection chamber 26 of the collection module 14.

Referring to Figs. 15-17, use of a biological fluid collection system 10 of the present disclosure having a collection module 14 and a power source 506 will now be described. Use of the embodiment illustrated in Figs. 15-17 involves similar steps of use as the embodiment illustrated in Figs. 4A-6C, as described in detail above. For the sake of brevity, these similar steps of using a biological fluid collection system 10 of the present disclosure having a collection module 14 and a power source 506 will not all be discussed in conjunction with the embodiment illustrated in Figs. 15-17.

In use, as described above, a needle cannula 100 (Figs. 28 and 29) is inserted into the passageway 36 of the housing 20 of the collection module 14 through the inlet port 32, such as through the pierceable self-sealing stopper 82 of closure 28. As described above, in one embodiment, the biological fluid collection system 10 including the collection module 14 and the power source 506 may be inserted into a conventional tube holder 102 having a cannula 100 through which biological fluid, such as a blood sample 12, is passed.

When a user desires to pull a blood sample 12 into the collection module 14 from the conventional tube holder 102 by the draw of a vacuum created within the power source 506, the user moves the plunger 514 away from the first end 522 of the barrel 510 to move the stopper to the second stopper position (Fig. 16) thereby creating a vacuum that pulls the sample 12 within the collection chamber 26 of the collection module 14.

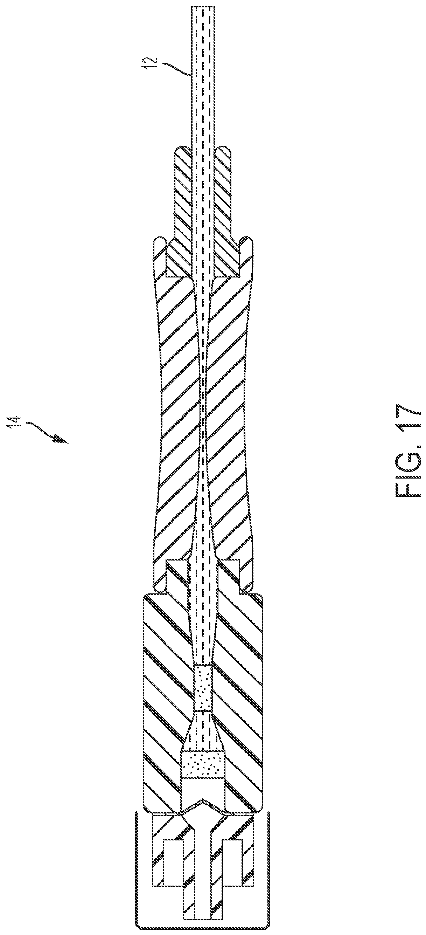

As described above, once sample collection is complete, the power source 506 and the collection module 14 are separated from the tube holder 102 (Fig. 29), and then the power source 506 is separated from the collection module 14 (Fig. 17).

Once the collection module 14 is separated from the power source 506, the cap 30 may then be removed from the collection module 14 (Fig. 26) exposing the outlet port 34 of the housing 20 of the collection module 14. Removal may be accomplished by the user grasping an exterior portion of the cap 30 and pulling the cap 30 from the housing 20. The blood sample 12 is held within the passageway 36 of the housing 20, e.g., the collection chamber 26, by capillary action after removal of the cap 30.

As described above, the blood sample 12 may then be dispensed from the collection module 14 by activation of the actuation portion 61 as shown in Figs. 18 and 19.

As described herein, the present disclosure provides a biological fluid collection system that includes a power source for a collection module that receives a sample and provides flow-through blood stabilization technology and a precise sample dispensing function for point-of-care and near patient testing applications. A power source of the present disclosure provides a user activated vacuum source for drawing a biological fluid sample within a collection module.

A collection module of the present disclosure is able to effectuate distributed mixing of a sample stabilizer within a blood sample and dispense the stabilized sample in a controlled manner. In this manner, a biological fluid collection system of the present disclosure enables blood micro-sample management, e.g., passive mixing with a sample stabilizer and controlled dispensing, for point-of-care and near patient testing applications.

Advantageously, a biological fluid collection system of the present disclosure provides a consistent blood sample management tool for point-of-care and near patient testing applications, automatic blood draw, passive mixing technology, and controlled small sample dispensing capability to point-of-care cartridge and standard luer interfaces with near patient testing receiving ports.

While this disclosure has been described as having exemplary designs, the present disclosure can be further modified within the spirit and scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.
Further Aspects are:
Aspect 1. A biological fluid collection system, comprising:
   a collection module adapted to receive a sample, the collection module comprising:
      a housing having an inlet port and an outlet port, the inlet port and the outlet port in fluid communication;
      a mixing chamber disposed between the inlet port and the outlet port; and
      a collection chamber disposed between the mixing chamber and the outlet port, the collection chamber including an actuation portion, wherein the actuation portion is transitionable between a first position in which the sample is containable within the collection chamber and a second position in which a portion of the sample is expelled from the collection chamber;
   a power source removably connectable with the collection module, the power source creates a vacuum that draws the sample within the collection chamber, the power source comprising:
      a barrel in communication with the collection chamber, the barrel defining an interior and having a first end, a second end, and a sidewall therebetween;
      a piston slidably disposed within the interior of the barrel, the piston sized relative to the interior to provide sealing engagement with the sidewall of the barrel, the piston transitionable between a first piston position, in which the piston is a first distance from the first end of the barrel, and a second piston position, in which the piston is a second distance from the first end of the barrel, the second distance greater than the first distance; and
      a spring disposed between the first end of the barrel and the piston.
Aspect 2. The biological fluid collection system of aspect 1, wherein the power source further comprises:
   an activation button disposed on a portion of the barrel; and
   a lock in communication with the spring and the activation button, the lock transitionable between a locked position, in which the lock locks the piston in the first piston position and maintains the spring in a compressed position, and an unlocked position, in which the piston is unlocked and the spring is permitted to drive the piston to the second piston position thereby creating a vacuum that draws the sample within the collection chamber,
   wherein actuation of the activation button moves the lock to the unlocked position.
Aspect 3. The biological fluid collection system of aspect 1, wherein the barrel is removably connectable with a portion of the collection module.
Aspect 4. The biological fluid collection system of aspect 1, wherein the collection module further comprises:
   a sample stabilizer disposed between the inlet port and the mixing chamber; and
   a cap having a venting plug, the cap seals the outlet port, wherein the venting plug allows air to pass therethrough and prevents the sample from passing therethrough.
Aspect 5. The biological fluid collection system of aspect 4, further comprising:
   a material including pores disposed between the inlet port and the mixing chamber; and
   a sample stabilizer within the pores of the material.
Aspect 6. The biological fluid collection system of aspect 5, wherein the material is an open cell foam and the sample stabilizer is a dry anticoagulant powder.
Aspect 7. The biological fluid collection system of aspect 1, further comprising a closure covering the inlet port.
Aspect 8. A biological fluid collection system, comprising:
   a collection module adapted to receive a sample, the collection module comprising:
      a housing having an inlet port and an outlet port, the inlet port and the outlet port in fluid communication;
      a mixing chamber disposed between the inlet port and the outlet port; and
      a collection chamber disposed between the mixing chamber and the outlet port, the collection chamber including an actuation portion, wherein the actuation portion is transitionable between a first position in which the sample is containable within the collection chamber and a second position in which a portion of the sample is expelled from the collection chamber;
   a power source removably connectable with the collection module, the power source having a vacuum that draws the sample within the collection chamber, the power source comprising:
      a spike in communication with the collection chamber;
      an evacuated tube having a first tube end, a second tube end, and a sidewall extending therebetween and defining a tube interior, the evacuated tube containing the vacuum; and
      a closure sealing the first tube end,
      wherein, with the evacuated tube engaged with the spike such that a portion of the spike pierces the closure and enters the tube interior, the vacuum of the evacuated tube draws the sample within the collection chamber.
Aspect 9. The biological fluid collection system of aspect 8, wherein the power source further comprises a tube holder removably connectable with a portion of the collection module, the tube holder defining an interior and having a first end, a second end, and a tube holder sidewall therebetween.
Aspect 10. The biological fluid collection system of aspect 8, wherein the evacuated tube is movably disposed within the interior of the tube holder between a first tube position, in which the evacuated tube is disengaged from the spike, and a second tube position, in which the closure of the evacuated tube is pierced by the spike.
Aspect 11. The biological fluid collection system of aspect 8, wherein, with the evacuated tube in the first tube position, a portion of the second tube end is exposed from the second end of the tube holder and the second tube end can be pushed to move the evacuated tube to the second tube position.
Aspect 12. The biological fluid collection system of aspect 8, wherein the collection module further comprises:
   a sample stabilizer disposed between the inlet port and the mixing chamber; and
   a cap having a venting plug, the cap seals the outlet port, wherein the venting plug allows air to pass therethrough and prevents the sample from passing therethrough.
Aspect 13. The biological fluid collection system of aspect 8, further comprising:
   a material including pores disposed between the inlet port and the mixing chamber; and
   a sample stabilizer disposed within the pores of the material.
Aspect 14. The biological fluid collection system of aspect 13, wherein the material is an open cell foam and the sample stabilizer is the dry anticoagulant powder.
Aspect 15. The biological fluid collection system of aspect 8, further comprising a collection module closure covering the inlet port.
Aspect 16. A biological fluid collection system, comprising:
   a collection module adapted to receive a sample, the collection module comprising:
      a housing having an inlet port and an outlet port, the inlet port and the outlet port in fluid communication;
      a mixing chamber disposed between the inlet port and the outlet port; and
      a collection chamber disposed between the mixing chamber and the outlet port, the collection chamber including an actuation portion, wherein the actuation portion is transitionable between a first position in which the sample is containable within the collection chamber and a second position in which a portion of the sample is expelled from the collection chamber;
   a power source removably connectable with the collection module, the power source creates a vacuum that draws the sample within the collection chamber, the power source comprising:
      a barrel in communication with the collection chamber, the barrel defining an interior and having a first end, a second end, and a sidewall therebetween;
      a stopper slidably disposed within the interior of the barrel, the stopper sized relative to the interior to provide sealing engagement with the sidewall of the barrel, the stopper transitionable between a first stopper position, in which the stopper is a first distance from the first end of the barrel, and a second stopper position, in which the stopper is a second distance from the first end of the barrel, the second distance greater than the first distance; and
      a plunger having a first plunger end and a second plunger end, a portion of the first plunger end engaged with the stopper, wherein movement of the plunger away from the first end of the barrel moves the stopper to the second stopper position thereby creating a vacuum that draws the sample within the collection chamber.
Aspect 17. The biological fluid collection system of aspect 16, wherein the collection module further comprises:
   a sample stabilizer disposed between the inlet port and the mixing chamber; and
   a cap having a venting plug, the cap seals the outlet port, wherein the venting plug allows air to pass therethrough and prevents the sample from passing therethrough.
Aspect 18. The biological fluid collection system of aspect 16, further comprising:
   a material including pores disposed between the inlet port and the mixing chamber; and
   a sample stabilizer disposed within the pores of the material.
Aspect 19. The biological fluid collection system of aspect 18, wherein the material is an open cell foam and the sample stabilizer is a dry anticoagulant powder.
Aspect 20. The biological fluid collection system of aspect 16, further comprising a closure covering the inlet port.

## Claims

1. A biological fluid collection system, comprising:
a collection module adapted to receive a sample, the collection module comprising:
a housing having an inlet port and an outlet port, the inlet port and the outlet port in fluid communication;
a mixing chamber disposed between the inlet port and the outlet port; and
a collection chamber disposed between the mixing chamber and the outlet port, the collection chamber including an actuation portion, wherein the actuation portion is transitionable between a first position in which the sample is containable within the collection chamber and a second position in which a portion of the sample is expelled from the collection chamber;
a power source removably connectable with the collection module, the power source having a vacuum that draws the sample within the collection chamber, the power source comprising:
a spike in communication with the collection chamber;
an evacuated tube having a first tube end, a second tube end, and a sidewall extending therebetween and defining a tube interior, the evacuated tube containing the vacuum; and
a closure sealing the first tube end,
wherein, with the evacuated tube engaged with the spike such that a portion of the spike pierces the closure and enters the tube interior, the vacuum of the evacuated tube draws the sample within the collection chamber.

2. The biological fluid collection system of claim 1, wherein the power source further comprises a tube holder removably connectable with a portion of the collection module, the tube holder defining an interior and having a first end, a second end, and a tube holder sidewall therebetween.

3. The biological fluid collection system of claim 2, wherein the evacuated tube is movably disposed within the interior of the tube holder between a first tube position, in which the evacuated tube is disengaged from the spike, and a second tube position, in which the closure of the evacuated tube is pierced by the spike.

4. The biological fluid collection system of claim 3, wherein, with the evacuated tube in the first tube position, a portion of the second tube end is exposed from the second end of the tube holder and the second tube end can be pushed to move the evacuated tube to the second tube position.

5. The biological fluid collection system of claim 1, wherein the collection module further comprises:
a sample stabilizer disposed between the inlet port and the mixing chamber; and
a cap having a venting plug, the cap seals the outlet port, wherein the venting plug allows air to pass therethrough and prevents the sample from passing therethrough.

6. The biological fluid collection system of claim 1, further comprising:
a material including pores disposed between the inlet port and the mixing chamber; and
a sample stabilizer disposed within the pores of the material.

7. The biological fluid collection system of claim 6, wherein the material is an open cell foam and the sample stabilizer is the dry anticoagulant powder.

8. The biological fluid collection system of claim 1, further comprising a collection module closure covering the inlet port.

9. The biological fluid collection system of claim 1, wherein the collection module is configured to be directly connectable to a luer line.

10. A biological fluid collection system, comprising:
a collection module adapted to receive a sample, the collection module comprising:
a housing having an inlet port and an outlet port, the inlet port and the outlet port in fluid communication;
a mixing chamber disposed between the inlet port and the outlet port; and
a collection chamber disposed between the mixing chamber and the outlet port, the collection chamber including an actuation portion, wherein the actuation portion is transitionable between a first position in which the sample is containable within the collection chamber and a second position in which a portion of the sample is expelled from the collection chamber;
a power source removably connectable with the collection module, the power source creates a vacuum that draws the sample within the collection chamber, the power source comprising:
a barrel in communication with the collection chamber, the barrel defining an interior and having a first end, a second end, and a sidewall therebetween;
a stopper slidably disposed within the interior of the barrel, the stopper sized relative to the interior to provide sealing engagement with the sidewall of the barrel, the stopper transitionable between a first stopper position, in which the stopper is a first distance from the first end of the barrel, and a second stopper position, in which the stopper is a second distance from the first end of the barrel, the second distance greater than the first distance; and
a plunger having a first plunger end and a second plunger end, a portion of the first plunger end engaged with the stopper, wherein movement of the plunger away from the first end of the barrel moves the stopper to the second stopper position thereby creating a vacuum that draws the sample within the collection chamber.

11. The biological fluid collection system of claim 10, wherein the collection module further comprises:
a sample stabilizer disposed between the inlet port and the mixing chamber; and
a cap having a venting plug, the cap seals the outlet port, wherein the venting plug allows air to pass therethrough and prevents the sample from passing therethrough.

12. The biological fluid collection system of claim 10, further comprising:
a material including pores disposed between the inlet port and the mixing chamber; and
a sample stabilizer disposed within the pores of the material.

13. The biological fluid collection system of claim 12, wherein the material is an open cell foam and the sample stabilizer is a dry anticoagulant powder.

14. The biological fluid collection system of claim 10, further comprising a closure covering the inlet port.

15. The biological fluid collection system of claim 10, wherein the collection module is configured to be directly connectable to a luer line.

16. The biological fluid collection system of claim 10, wherein
the power source has an activation button and is configured to automatically create a vacuum that draws the sample within the collection chamber upon actuation of the activation button, and wherein the barrel is removably connectable with a portion of the collection module.
